# EUROPEAN PATENT APPLICATION

(11) **EP 4 818 173 A1**
(43) Date of publication of application: **07.10.2026**
(21) Application number: 25168121.9
(22) Date of filing: 02.04.2025
(51) Int. Cl.: C07D 493/08

(54) **OXYGEN CONTAINING BRIDGED BICYCLIC COMPOUNDS AS VERSATILE SOLVENTS**

(71) Applicant: ECOLE POLYTECHNIQUE FEDERALE DE LAUSANNE (EPFL), 1015 Lausanne (CH)
(72) Inventor: NELIS, Tom, 1110 Morges (CH); BEHAGHEL DE BUEREN, Jean, 1005 Lausanne (CH); LUTERBACHER, Jeremy Scott, 1022 Chavannes-Renens (CH)
(74) Representative: Vossius & Partner Patentanwälte Rechtsanwälte mbB

(57) **Abstract**

The invention provides bridged, bicyclic compounds selected from a trioxabicycloheptane, a trioxabicyclooctane, a trioxabicyclononane, and from alkylated and/or alkoxylated derivatives of any of these compounds as a new class of solvents useful for the preparation of solutions and dispersion for diverse applications. Moreover, methods making use of these compounds as solvents, solutions and dispersions containing these compounds, and solvent formulations containing these compounds are provided.

## Description

The invention provides bridged, bicyclic compounds selected from a trioxabicycloheptane, a trioxabicyclooctane, a trioxabicyclononane, and from alkylated and/or alkoxylated derivatives of any of these compounds as a new class of solvents useful for the preparation of solutions and dispersion for diverse applications. Moreover, methods making use of these compounds as solvents, solutions and dispersions containing these compounds, and solvent formulations containing these compounds are provided.

Solvents are used in various chemical processes, e.g., to provide a medium for molecules to meet and react, to extract species from a solid or liquid mixture, for the purification of species of interest, or to allow a substance of interest to be processed in a dissolved or dispersed form. They often end up as the major component of a reaction mixture or as the major substance used in a process. As a matter of fact, the American Chemical Society has reported that solvents generally represent 50 to 80% of the total mass used in a typical batch operation.

In addition to their fundamental functional roles, solvents have long been shown to have a significant impact on reactions kinetics, chemical equilibria, or UV/Vis absorption spectra. Therefore, the choice of an appropriate solvent in which to perform a reaction is of importance. A good solvent should solubilize the reactive species, be stable in the process conditions and in presence of the other elements of the reactive mixture, while enhancing process performance towards the highest productivity. Considering this, dipolar aprotic solvents are widely used in chemistry thanks to their ability to dissolve a broad variety of substances, including salts, their non-hydrogen bond donating nature and their relatively low cost. Notably, dipolar aprotic solvents are particularly well suited for SN₂ and SN_{Ar} reactions as they do not form hydrogen bonds with nucleophiles, and have been shown to greatly enhance the rate of these reactions. For these reasons, dipolar aprotic solvents are also indispensable in the chemical industry. Similarly, ethereal solvents are greatly appreciated, despite of their reduced polarity, for their hydrogen-bond accepting nature, which allows them to coordinate reactive species.

The development of viable replacements for conventional polar aprotic solvents (PAS) has recently been identified as a key green chemistry research area according to ACS Green Chemistry Institute^{®} Pharmaceutical Roundtable. This class of organic solvents is considered as one of the most difficult to replace solvents due to their unique solubilizing properties and the ability to provide strong interactions with the active pharmaceutical ingredients. Research in this field is also highly driven by regulatory initiatives. For instance, European Union REACH (Registration, Evaluation, Authorisation and Restriction of Chemicals) recently has restricted the industrial use of N-methylpyrrolidone (NMP), and listed others of the most common PAS like N,N-dimethylformamide (DMF), N,N-dimethylacetamide (DMAc) as substances of very high concern due to their severe reproductive toxicity.

In the last decade, the abovementioned environment, health and safety (EHS) considerations have greatly increased research interest towards sustainable replacements for conventional solvents. This led to the emergence in literature of dozens of candidates. Sulfolane was originally considered to be an attractive alternative to dipolar aprotic solvents. However, sulfolane was later found to be reprotoxic and is now ranked as "hazardous" in CHEM21 solvent selection guide. Another compound that is currently being up-scaled is dihydrolevoglucosenone (Cyrene). Cyrene is a bio-based molecule that can be prepared in two steps from cellulose that has similar solvation properties as traditional NMP. Two other promising bio-based solvents are γ-valerolactone (GVL) and 2-methyltetrahydrofuran (2-MeTHF). The structures of solvents discussed above, along with some other relevant proposed solvent replacements, are shown in the following:

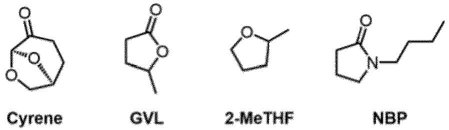

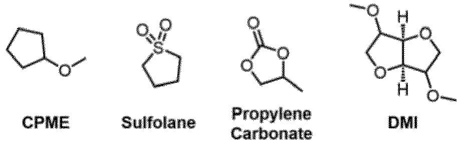

In spite of the research efforts on dipolar aprotic and ethereal solvents, and the vast library of potential alternatives to date, problematic traditional solvents remain very popular for industrial applications. Indeed, Cyrene suffers from reduced versatility due to its high reactivity. Similarly, GVL was shown to be prone to ring-opening reactions. Furthermore, these alternatives leave room for improvement from an environmental point of view, as their high boiling points limit their recyclability, or would make it energy-intensive.

Therefore, in the area of dipolar aprotic and ethereal solvents, there is still a pressing need for new candidates which ideally combine as many as possible of the following characteristics: affordable, non-toxic, bio-based, bio-degradable, stable and recyclable.

Glycerol (propane-1,2,3-triol, glycerine) is a non-toxic, colorless and odorless viscous liquid, and is produced as co-product of the bio-diesel manufacturing process. Glycerol is stoichiometrically produced for each triglyceride molecule processed to bio-diesel, regardless of the nature of the triglyceride used. Because of its low toxicity, large availability, low price, and versatility, glycerol appears to be a sustainable material that is suitable for various applications. For example, crude glycerol is currently being used as fuel or in food products, or for the manufacturing of value-added chemicals such as polymers, cosmetics, fuel additives or lubricants. Due to the exponential growth in the production of bio-diesel in recent years, glycerol has been oversupplied, and its production has surpassed two million metric tons, which could cause environmental issues. In addition, its value has declined steadily. For economic competitiveness of the bio-diesel industry, increasing interest has been stimulated by research on the conversion of glycerol into value-added chemicals.

### Summary

According to a first aspect, the invention provides the use of a bridged, bicyclic compound selected from a trioxabicycloheptane, a trioxabicyclooctane, a trioxabicyclononane, and from alkylated and/or alkoxylated derivatives of any of these compounds, as a solvent. As shall be explained in further detail below, the solvent is suitable for dissolving or dispersing a broad variety of substances in diverse applications. As examples, reference can be made to the use as a solvent for a chemical reaction, as a process solvent, e.g. to facilitate the mixing of substances or the transfer of substances during a process, as a solvent in polymer production and/or processing, e.g. for solution spinning, as an extraction solvent, as a solvent for purifying a substance, e.g. by recrystallization, as a solvent for cleaning, as a solvent for the formulation and/or application of an active agent, e.g. an agrochemical agent, or as a solvent for the storage of a substance.

According to a related second aspect, a method is provided for the preparation of a solution or dispersion, which method comprises dissolving or dispersing a substance comprising a step of dissolving or dispersing a substance in a single solvent or in a solvent formulation, wherein the single solvent is a bridged, bicyclic compound selected from a trioxabicycloheptane, a trioxabicyclooctane, a trioxabicyclononane, and from alkylated and/or alkoxylated derivatives of any of these compounds and wherein the solvent formulation comprises at least one type of the bridged, bicyclic compound as solvent(s). According to a third aspect, the invention provides a solution or dispersion, wherein a substance is dissolved or dispersed in a single solvent or in a solvent formulation, and wherein the single solvent is a bridged, bicyclic compound selected from a trioxabicycloheptane, a trioxabicyclooctane, a trioxabicyclononane, and from alkylated and/or alkoxylated derivatives of any of these compounds and the solvent formulation comprises at least one type of the bridged, bicyclic compound as solvent(s). A fourth aspect relates to solvent formulations comprising a bridged, bicyclic compound selected from a trioxabicycloheptane, a trioxabicyclooctane, a trioxabicyclononane, and from alkylated and/or alkoxylated derivatives of any of these compounds as a solvent in combination with a further solvent component.

According to a fifth aspect, a method is provided for the preparation of a trioxabicyclooctane or of an alkylated derivative thereof, which are suitable for use in the first to fourth aspect.

A summary of aspects of the invention and preferred embodiments thereof is provided by the following items.
1. Use of one or more types of a bridged, bicyclic compound selected from a trioxabicycloheptane, a trioxabicyclooctane, a trioxabicyclononane, and from alkylated and/or alkoxylated derivatives of any of these compounds, as solvent(s).
2. The use in accordance with item 1, wherein the one or more types of the bridged, bicyclic compound is/are selected from 2,5,7-trioxabicyclo[2.2.1]heptane, 3,6,8-trioxabicyclo[3.2.1]octane, 2,5,7-trioxabicyclo[2.2.2]octane, 3,7,9-trioxabicyclo[4.2.1]nonane, 3,8,9-trioxabicyclo[4.2.1]nonane, 3,7,9-trioxabicyclo[3.3.1]nonane, and from alkylated and/or alkoxylated derivatives of any of these compounds.
3. The use in accordance with item 1 or 2, wherein the alkylated and/or alkoxylated derivatives are derivatives carrying one or more substituents independently selected from methyl, ethyl, methoxy and ethoxy attached to one or more carbon ring members of the bridged, bicyclic ring system of the trioxabicycloheptane, the trioxabicyclooctane or the trioxabicyclononane.
4. The use in accordance with any one of items 1 to 3, wherein the one or more substituents is/are independently selected from methyl, ethyl, methoxy and ethoxy, with the proviso that the alkoxylated derivatives carry not more than one alkoxy substituent.
5. The use in accordance with any one of items 1 to 3, wherein the one or more types of the bridged, bicyclic compound is/are selected from compounds of the following formulae (A), (B), (C), (D), (E) or (F): 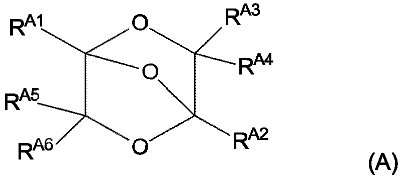
   wherein R^{A1} to R^{A6} are independently selected from hydrogen and methyl, and preferably
   R^{A1} to R^{A6} are all methyl, or
   R^{A1} and R^{A2} are methyl, R^{A3} and R^{A5} are methyl, and R^{A4} and R^{A6} are hydrogen, or
   R^{A1} and R^{A2} are methyl, and R^{A3} to R^{A6} are all hydrogen, or
   R^{A1} to R^{A6} are all hydrogen, or
   R^{A1} and R^{A2} are hydrogen, R^{A3} and R^{A5} are methyl, and R^{A4} and R^{A6} are hydrogen, or
   R^{A1} and R^{A2} are hydrogen, and R^{A3} to R^{A6} are methyl; 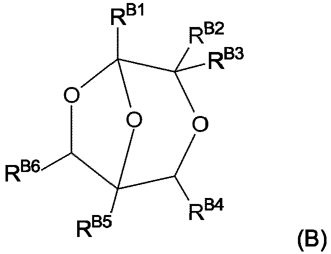
   wherein R^{B1} and R^{B3} to R^{B6} are independently selected from hydrogen and methyl, and R^{B2} is selected from hydrogen, methyl, methoxy and ethoxy, and preferably:
      R^{B1} to R^{B6} are all hydrogen, or
      R^{B1} is methyl and R^{B2} to R^{B6} are hydrogen, or
      R^{B1} and R^{B2} are methyl and R^{B3} to R^{B6} are hydrogen, or
      R^{B1} to R^{B3} are methyl and R^{B4} to R^{B6} are hydrogen, or
      R^{B1} is hydrogen, R^{B2} is methyl and R^{B3} to R^{B6} are hydrogen, or
      R^{B1} to R^{B5} are hydrogen and R^{B6} is methyl, or
      R^{B1} and R^{B6} are methyl and R^{B2} to R^{B5} are hydrogen, or
      R^{B1} is hydrogen, R^{B2} is methyl, R^{B3} is methoxy and R^{B4} to R^{B6} are hydrogen, or
      R^{B1} is methyl, R^{B2} is methoxy and R^{B3} to R^{B6} are hydrogen, or
      R^{B1} is methyl, R^{B2} is ethoxy and R^{B3} to R^{B6} are hydrogen, or
      R^{B1} is methyl, R^{B2} is methoxy, R^{B3} is methyl and R^{B4} to R^{B6} are hydrogen, or
      R^{B1} is methyl, R^{B2} is ethoxy, R^{B3} is methyl and R^{B4} to R^{B6} are hydrogen, or
      R^{B1} is hydrogen, R^{B2} and R^{B3} are methyl and R^{B4} to R^{B6} are hydrogen; or
      R^{B1} is hydrogen, R^{B2} and R^{B3} are methyl, R^{B4} is hydrogen, R^{B5} is methyl and R^{B6} is hydrogen; 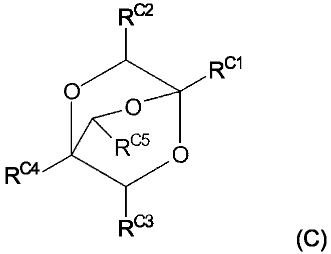
   wherein R^{C1} to R^{C5} are independently selected from hydrogen and methyl, and preferably:
      R^{C1} to R^{C5} are all hydrogen, or
      R^{C1} is hydrogen, R^{C2} is methyl, and R^{C3} to R^{C5} are hydrogen, or
      R^{C1} and R^{C2} are hydrogen, R^{C3} is methyl, and R^{C4} and R^{C5} are hydrogen;
      R^{C1} is methyl and R^{C2} to R^{C5} are hydrogen, or
      R^{C1} and R^{C2} are methyl and R^{C3} to R^{C5} are hydrogen; 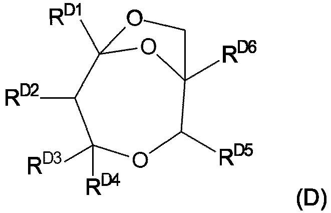
   wherein R^{D1} is selected from hydrogen, methyl and ethyl and R^{D2} to R^{D6} are independently selected from hydrogen and methyl, and preferably:
      R^{D1} to R^{D6} are hydrogen, or
      R^{D1} is methyl, and R^{D2} to R^{D6} are hydrogen, or
      R^{D1} is ethyl and R^{D2} to R^{D6} are hydrogen, or
      R^{D1} and R^{D2} are methyl and R^{D3} to R^{D6} are hydrogen, or
      R^{D1} is methyl, R^{D2} to R^{D5} are hydrogen and R^{D6} is methyl, or
      R^{D1} is methyl, R^{D2} to R^{D4} are hydrogen, R^{D5} is methyl and R^{D6} is hydrogen, or
      R^{D1} is methyl, R^{D2} hydrogen, R^{D3} methyl, R^{D4} and R^{D5} hydrogen and R^{D6} is methyl, or
      R^{D1} is methyl, R^{D2} is hydrogen, R^{D3} and R^{D4} is methyl, R^{D5} is hydrogen and R^{D6} is methyl; 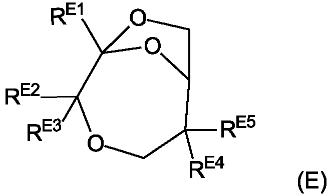
   wherein R^{E1} to R^{E5} are independently selected from hydrogen and methyl, and preferably:
      R^{E1} to R^{E5} are all hydrogen, or
      R^{E1} is methyl and R^{E2} to R^{E5} are hydrogen, or
      R^{E1} to R^{E3} are hydrogen and R^{E4} and R^{E5} are methyl, or
      R^{E1} is methyl, R^{E2} and R^{E3} are hydrogen and R^{E4} and R^{E5} are methyl, or
      R^{E1} and R^{E2} are both methyl and R^{E3} to R^{E5} are hydrogen, or
      R^{E1} to R^{E3} are methyl and R^{E4} and R^{E5} are hydrogen, or
      R^{E1} and R^{E2} are methyl, R^{E3} is hydrogen and R^{E4} and R^{E5} are methyl, or
      R^{E1} to R^{E5} are all methyl; 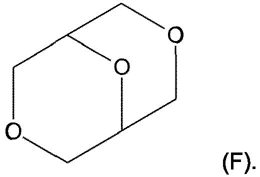
6. The use in accordance with any one of items 1 to 5, wherein one type of the bridged, bicyclic compound is used as a single solvent.
7. The use in accordance with any one of items 1 to 5 wherein the one or more types of the bridged, bicyclic compound is/are used as solvent(s) comprised by a solvent formulation.
8. The use in accordance with item 7, wherein the solvent formulation comprises the one or more types of the bridged, bicyclic compound at total concentration of 20 wt% or more, more preferably 50 wt% or more, based on the total weight of the solvent formulation.
9. The use in accordance with item 7 or 8, wherein the solvent formulation is a solvent mixture comprising or consisting of two or more, preferably two, types of the bridged, bicyclic compounds.
10. The use in accordance with any one of items 7 to 9, wherein the solvent formulation is a solvent mixture comprising or consisting of one or more types of the bridged, bicyclic compound mixed with at least one further solvent which is not a bridged, bicyclic compound selected from a trioxabicycloheptane, a trioxabicyclooctane, a trioxabicyclononane, and from alkylated and/or alkoxylated derivatives of any of these compounds.
11. The use in accordance with item 10, wherein the at least one further solvent is selected from water, nitriles, esters, ethers, alcohols, hydrocarbons, ketones, halogenated hydrocarbons and from combinations thereof.
12. The use in accordance with any one of items 9 to 11, wherein the solvent mixture contains the one or more types of the bridged, bicyclic compound at a total concentration of 80 wt% or more, more preferably 90 wt% or more, based on the total weight of the solvent mixture.
13. The use in accordance with item 7 or 8, wherein the solvent formulation comprises a eutectic mixture of the one or more types of the bridged, bicyclic compound and at least one hydrogen bond donor compound.
14. The use in accordance with any one of items 1 to 13, wherein the one or more types of the bridged, bicyclic compound is/are used as solvent(s) for a chemical reaction, as process solvent(s), as solvent(s) in polymer production and/or processing, as extraction solvent(s), as solvent(s) for purifying a substance, as solvent(s) for cleaning, as solvent(s) for the formulation and/or application of an active agent, or as solvent(s) for the storage of a substance.
15. The use in accordance with item 14, wherein the one or more types of the bridged, bicyclic compound is/are used as solvent(s) for a chemical reaction, preferably wherein the chemical reaction is an organic synthesis reaction.
16. The use in accordance with item 15, wherein the chemical reaction is selected from a cross-coupling reaction, an alkylation reaction, a hydrogenation reaction, an enzymatic reaction, an amidation reaction (amide bond formation reaction), an esterification reaction (ester bond formation reaction), an aromatic nucleophilic substitution, an acylation, a Grignard reagent formulation, and a polymerization reaction.
17. The use in accordance with any one of items 1 to 14, wherein the one or more types of the bridged, bicyclic compound is/are used as solvent(s) in peptide synthesis.
18. The use in accordance with any one of items 1 to 14, wherein the one or more types of the bridged, bicyclic compound is/are used as solvent(s) in solution spinning.
19. The use in accordance with any one of items 1 to 18, wherein the one or more types of the bridged, bicyclic compound are used in a solution or dispersion wherein a substance is dissolved or dispersed which comprises at least one of the following (i) to (iv):
   (i) at least one type of polymer, such as a polyacrylate, a polymethacrylate (such as poly(methyl methacrylate)), a polyether, a polyurea, a polyacetal, a polyolefin, a polysulfone, a polyamide, a polyimide, a polyester (such as lactic acid or glycolic acid), polystyrene, polyacrylonitrile, polyvinylchloride, polyvinylpyrrolidone, polyvinylacetate, a polycarbonate, a polyurethane, an epoxy resin, a cellulose derivative, or a copolymer combining appropriate units of two or more of these exemplary polymers;
   (ii) at least one type of a monomer, such as a monomer suitable for the formation of an exemplary polymer or copolymer as referred to in (i);
   (iii) at least one type of organic chemical reactant, such as an amino acid or a derivative thereof carrying one or more protecting groups, e.g. a protecting group for an amino group;
   (iv) at least one type of an active agent, preferably an agrochemical active agent, such as an insecticide, a herbicide, a fungicide, or a pesticide.
20. A method for the preparation of a solution or dispersion, comprising a step of dissolving or dispersing a substance in a single solvent or in a solvent formulation, wherein the single solvent is a bridged, bicyclic compound selected from a trioxabicycloheptane, a trioxabicyclooctane, a trioxabicyclononane, and from alkylated and/or alkoxylated derivatives of any of these compounds and wherein the solvent formulation comprises one or more types of a bridged, bicyclic compound selected from a trioxabicycloheptane, a trioxabicyclooctane, a trioxabicyclononane, and from alkylated and/or alkoxylated derivatives of any of these compounds as solvent(s).
21. The method in accordance with item 20, wherein the bridged, bicyclic compounds are as defined in any one of items 1 to 5 above, and are more preferably selected from compounds 1 to 42 as defined herein.
22. The method in accordance with item 20 or 21, wherein the solvent formulation comprises the one or more types of the bridged, bicyclic compound at a total concentration of 20 wt% or more, more preferably 50 wt% or more, based on the total weight of the solvent formulation.
23. The method in accordance with any one of items 20 to 22, wherein the solvent formulation is a solvent mixture comprising or consisting of two or more, preferably two, types of the bridged, bicyclic compounds as solvents.
24. The method in accordance with item 20 to 23, wherein the solvent formulation is a solvent mixture comprising or consisting of one or more types of the bridged, bicyclic compound mixed with at least one further solvent which is not a bridged, bicyclic compound selected from a trioxabicycloheptane, a trioxabicyclooctane, a trioxabicyclononane, and from alkylated and/or alkoxylated derivatives of any of these compounds.
25. The method in accordance with item 24, wherein the at least one further solvent is selected from water, nitriles, esters, ethers, alcohols, hydrocarbons, ketones, halogenated hydrocarbons and from combinations thereof.
26. The method in accordance with any one of items 23 to 25, wherein the solvent mixture contains the one or more types of the bridged, bicyclic compound at a total concentration of 80 wt% or more, preferably 90 wt% or more, based on the total weight of the solvent mixture.
27. The method in accordance with any one of items 20 to 22, wherein the solvent formulation comprises or consists of a eutectic mixture of the one or more types of the bridged, bicyclic compound and at least one hydrogen bond donor compound.
28. The method in accordance with any one of items 20 to 27, wherein the dissolved or dispersed substance is dissolved or dispersed at a concentration of 0.03 g or more per g of the solution or dispersion.
29. The method in accordance with any one of items 20 to 28, wherein the dissolved or dispersed substance comprises at least one of the following (i) to (iv):
   (i) at least one type of polymer, such as a polyacrylate, a polymethacrylate (such as poly(methyl methacrylate)), a polyether, a polyurea, a polyacetal, a polyolefin, a polysulfone, a polyamide, a polyimide, a polyester (such as lactic acid or glycolic acid), polystyrene, polyacrylonitrile, polyvinylchloride, polyvinylpyrrolidone, polyvinylacetate, a polycarbonate, a polyurethane, an epoxy resin, a cellulose derivative, or a copolymer combining appropriate units of two or more of these exemplary polymers;
   (ii) at least one type of a monomer, such as a monomer suitable for the formation of an exemplary polymer or copolymer as referred to in (i);
   (iii) at least one type of organic chemical reactant, such as an amino acid or a derivative thereof carrying one or more protecting groups, e.g. a protecting group for an amino group;
   (iv) at least one type of an active agent, preferably an agrochemical active agent, such as an insecticide, a herbicide, a fungicide, or a pesticide.
30. The method in accordance with any one of items 20 to 29, wherein the preparation of the solution or dispersion forms part of a process for carrying out a chemical reaction, a process for the production of a polymer, a process for the preparation of a polymer coating, a polymer film or a polymer membrane, a solution spinning process, a solid phase peptide synthesis,or a process for the formulation or the application of an active agent, e.g. an agrochemical agent.
31. A solution or dispersion, comprising a substance which is dissolved or dispersed in a single solvent or in a solvent formulation, wherein the single solvent is a bridged, bicyclic compound selected from a trioxabicycloheptane, a trioxabicyclooctane, a trioxabicyclononane, and from alkylated and/or alkoxylated derivatives of any of these compounds and wherein the solvent formulation comprises one or more types of a bridged, bicyclic compound selected from a trioxabicycloheptane, a trioxabicyclooctane, a trioxabicyclononane, and from alkylated and/or alkoxylated derivatives of any of these compounds as solvent(s).
32. The solution or dispersion in accordance with item 31, wherein the bridged, bicyclic compounds are as defined in any one of items 1 to 5 above, and are more preferably selected from compounds 1 to 42 as defined herein.
33. The solution or dispersion in accordance with item 31 or 32, wherein the solvent formulation comprises the one or more types of the bridged, bicyclic compound at a total concentration of 20 wt% or more, more preferably 50 wt% or more, based on the total weight of the solvent formulation.
34. The solution or dispersion in accordance with any one of items 31 to 33, wherein the solvent formulation is a solvent mixture comprising or consisting of two or more, preferably two, types of the bridged, bicyclic compounds as solvents.
35. The solution or dispersion in accordance with item 31 to 36, wherein the solvent formulation is a solvent mixture comprising or consisting of one or more types of the bridged, bicyclic compound as solvent(s) mixed with at least one further solvent which is not a bridged, bicyclic compound selected from a trioxabicycloheptane, a trioxabicyclooctane, a trioxabicyclononane, and from alkylated and/or alkoxylated derivatives of any of these compounds.
36. The solution or dispersion in accordance with item 35, wherein the at least one further solvent is selected from water, nitriles, esters, ethers, alcohols, hydrocarbons, ketones, halogenated hydrocarbons and from combinations thereof.
37. The solution or dispersion in accordance with any one of items 34 to 36, wherein the solvent mixture contains the one or more types of the bridged, bicyclic compound at a total concentration of 80 wt% or more, preferably 90 wt% or more, based on the total weight of the solvent mixture.
38. The solution or dispersion in accordance with item 31 or 32, wherein the solvent formulation comprises or consists of a eutectic mixture of the one or more types of the bridged, bicyclic compound and at least one hydrogen bond donor compound.
39. The solution or dispersion in accordance with any one of items 31 to 38, wherein the dissolved or dispersed substance is dissolved or dispersed at a concentration of 0.03 g or more per g of the solution or dispersion.
40. The solution or dispersion in accordance with any one of items 31 to 39, wherein the dissolved or dispersed substance comprises at least one of the following (i) to (iv):
   (i) at least one type of polymer, such as a polyacrylate, a polymethacrylate (such as poly(methyl methacrylate)), a polyether, a polyurea, a polyacetal, a polyolefin, a polysulfone, a polyamide, a polyimide, a polyester (such as lactic acid or glycolic acid), polystyrene, polyacrylonitrile, polyvinylchloride, polyvinylpyrrolidone, polyvinylacetate, a polycarbonate, a polyurethane, an epoxy resin, a cellulose derivative, or a copolymer combining appropriate units of two or more of these exemplary polymers;
   (ii) at least one type of a monomer, such as a monomer suitable for the formation of an exemplary polymer or copolymer as referred to in (i);
   (iii) at least one type of organic chemical reactant, such as an amino acid or a derivative thereof carrying one or more protecting groups, e.g. a protecting group for an amino group;
   (iv) at least one type of an active agent, preferably an agrochemical active agent, such as an insecticide, a herbicide, a fungicide, or a pesticide.
41. A solvent formulation comprising one or more types of a bridged, bicyclic compound selected from a trioxabicycloheptane, a trioxabicyclooctane, a trioxabicyclononane, and from alkylated and/or alkoxylated derivatives of any of these compounds at a total concentration of 20 wt% or more, preferably 50 wt% or more, based on the total weight of the solvent formulation, which solvent formulation is either
   (i) a solvent mixture wherein the one or more types of a bridged, bicyclic compound is/are mixed with at least one further solvent which is not a bridged, bicyclic compound selected from a trioxabicycloheptane, a trioxabicyclooctane, a trioxabicyclononane, and from alkylated and/or alkoxylated derivatives of any of these compounds, or
   (ii) a eutectic mixture wherein the one or more types of a bridged, bicyclic compound are mixed with at least one hydrogen bond donor compound.
42. The solvent formulation in accordance with item 41, wherein the bridged, bicyclic compounds are as defined in any one of items 1 to 5 above, and are more preferably selected from compounds 1 to 42 as defined herein.
43. The solvent formulation in accordance with items 41 or 42, which is a solvent mixture wherein the at least one further solvent is selected from water, nitriles, esters, ethers, alcohols, hydrocarbons, ketones, halogenated hydrocarbons and from combinations thereof.
44. The solvent formulation in accordance with any one of items 41 to 43, which is a solvent mixture and which contains the one or more types of the bridged, bicyclic compound at a total concentration of 80 wt% or more, preferably 90 wt% or more, based on the total weight of the solvent mixture.
45. The solvent formulation in accordance with any one of items 41 to 44, which is a solvent mixture and which contains the at least one further solvent at a concentration of 1wt% or more, based on the total weight of the solvent mixture.
46. A method for the preparation of a trioxabicyclooctane or of an alkylated derivative thereof, said method comprising
   a) a step wherein a compound of formula (S1) 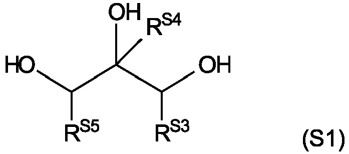 wherein R^{S3}, R^{S4} and R^{S5} are independently hydrogen or methyl, and are preferably all hydrogen,
      is reacted with a compound of formula (S2A) or a compound of formula (S2B) 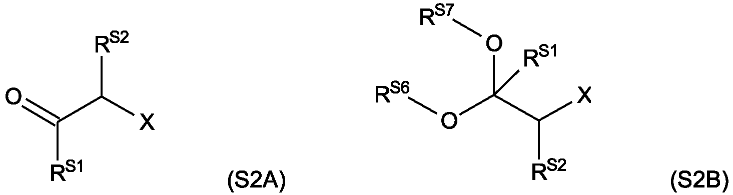 wherein
         X is Cl or Br, and is preferably Cl,
         R^{S1} and R^{S2} are independently hydrogen or methyl, preferably R^{S1} and R^{S2} are both hydrogen or R^{S1} is methyl and R^{S2} is hydrogen,
         R^{S6} and R^{S7} are C1-C3 alkyl, and are preferably both methyl;
      to obtain a mixture of isomer compounds (S3T), (S3C), (S4T) and (S4C), wherein X, R^{S1}, R^{S2}, R^{S3}, R^{S4} and R^{S5} are defined as above, 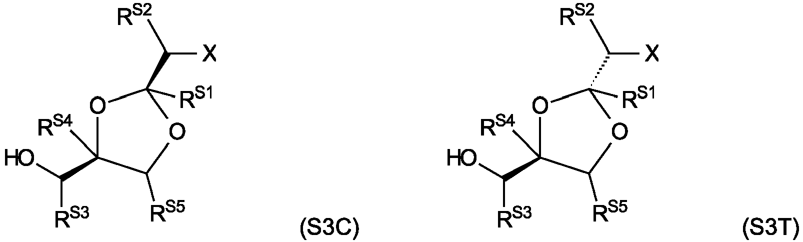 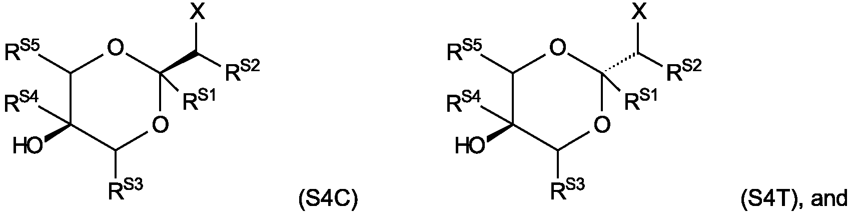
   b) a step wherein the compounds of formula (S3C) and (S4C) are subjected to intramolecular ether formation in the presence of NaOH or KOH, preferably NaOH, in an amount of 1 to 2.70 molar equivalents, relative to the total amount of compounds of formula (S3C) and (S4C), in water, to obtain the compounds of formula (S5) and (S6), wherein R^{S1}, R^{S2}, R^{S3}, R^{S4} and R^{S5} are as defined above: 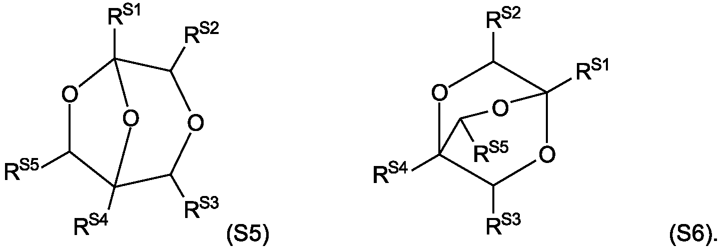
47. The method in accordance with item 46, wherein X is Cl and R^{S1} to R^{S5} are hydrogen, or R^{S1} is methyl and R^{S2} to R^{S5} are hydrogen.

### Detailed Disclosure

In the following, details on the aspects of the invention and their embodiments will be discussed.

### General Definitions

As noted above, the invention exploits, in its various aspects, the usefulness of specific bridged, bicyclic compounds which are selected from a trioxabicycloheptane, a trioxabicyclooctane, a trioxabicyclononane, and from alkylated and/or alkoxylated derivatives of any of these compounds, as solvents. It will be understood that any reference to "the bridged, bicyclic compounds" or to "these bridged, bicyclic compounds" herein is directed to the bridged, bicyclic compounds which are selected from a trioxabicycloheptane, a trioxabicyclooctane, a trioxabicyclononane, and from alkylated and/or alkoxylated derivatives of any of these compounds, including preferred types of these compounds as they are discussed, e.g., in the following. In order to facilitate the discussion, the abbreviated version "TBH" may be used herein to refer to a trioxabicycloheptane and to alkylated and/or alkoxylated derivatives thereof, the abbreviated version "TBO" may be used herein to refer to a trioxabicyclooctane and to alkylated and/or alkoxylated derivatives thereof, and the abbreviated version "TBN" may be used herein to refer to a trioxabicyclononane and to alkylated and/or alkoxylated derivatives thereof.

Bridged, bicyclic compounds which are selected from a trioxabicycloheptane, a trioxabicyclooctane, a trioxabicyclononane, and from alkylated and/or alkoxylated derivatives of any of these compounds, as well as their synthesis, are disclosed in the scientific literature. However, their suitability as solvents, in particular as polar, aprotic solvents, has not been appreciated.

The bridged, bicyclic compounds selected from a trioxabicycloheptane, a trioxabicyclooctane, a trioxabicyclononane, and from alkylated and/or alkoxylated derivatives of any of these compounds, have been found to be performant as solvents for diverse purposes, stable, and they can be conveniently recovered or recycled, e.g. by distillation. The experimental results obtained using the solvents described herein illustrate that these bridged, bicyclic compounds. benefit from a high to moderate polarity, and a high to moderate, ether like basicity. Without wishing to be bound by theory, it is considered that this allows them to exhibit high performance, *e*.*g*., as reaction solvents in reactions that are typically performed in dipolar aprotic solvents, as well as in reactions that are usually performed in less polar solvents (such as ethereal solvents) in which dipolar aprotic solvents generally underperform, and places them as a highly versatile solvents with a potential to exhibit high performance in many organic reactions. Moreover, starting products obtained from renewable resources can be relied on as for the provision of the bridged, bicyclic compounds. As examples, reference can be made to bio-based glycerol for the synthesis of TBO or TBN, bio-based acetoin or methyl acetoin for the synthesis of TBH and/or bio-based butane-1,2-4 triol for the synthesis of TBN.

As will be understood by the skilled reader, reference to more than one type of the bridged, bicyclic compounds is directed to types of compounds which differ in terms of their chemical structure, irrespective of whether the difference concerns the bridged, bicyclic ring system, or the structure, number or position of alkyl and/or alkoxy substituents which may be carried in by the bridged, bicyclic ring system in the alkylated and/or alkoxylated derivatives of the compounds.

In the context of the invention, the trioxabicycloheptane is preferably trioxabicyclo[2.2.1]heptane, and the alkylated and/or alkoxylated derivatives thereof are thus preferably alkylated and/or alkoxylated derivatives of this trioxabicycloheptane. The trioxabicyclooctane is preferably 3,6,8-trioxabicyclo[3.2.1]octane or 2,5,7-trioxabicyclo[2.2.2]octane, and the alkylated and/or alkoxylated derivatives thereof are thus preferably alkylated and/or alkoxylated derivatives of any of these trioxabicyclooctanes. The trioxabicyclononane is preferably 3,7,9-trioxabicyclo[4.2.1]nonane, 3,8,9-trioxabicyclo[4.2.1]nonane or 3,7,9-trioxabicyclo[3.3.1]nonane, and the alkylated and/or alkoxylated derivatives thereof are thus preferably alkylated and/or alkoxylated derivatives of any of these trioxabicyclononanes.

The alkylated and/or alkoxylated derivatives of the trioxabicycloheptane, trioxabicyclooctane, and trioxabicyclononane, are preferably methylated, ethylated, methoxylated and/or ethoxylated derivatives, i.e. derivatives wherein the trioxabicycloheptane, trioxabicyclooctane, or trioxabicyclononane ring system carries one or more substituents independently selected from methyl, ethyl, methoxy and ethoxy attached to one or more carbon ring members of the ring system of the trioxabicycloheptane, the trioxabicyclooctane or the trioxabicyclononane.

More preferably, the one or more substituents of the alkylated and/or alkoxylated derivatives is/are independently selected from methyl, ethyl, methoxy and ethoxy, with the proviso that the derivatives do not carry more than one alkoxy substituent.

Still more preferably, the one or more types of the bridged, bicyclic compound is/are selected from compounds of the following formulae (A), (B), (C), (D), (E) or (F): 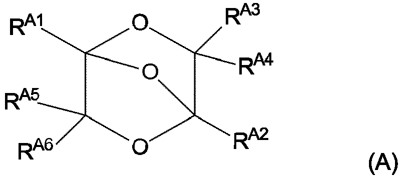
wherein R^{A1} to R^{A6} are independently selected from hydrogen and methyl, and preferably
   R^{A1} to R^{A6} are all methyl, or
   R^{A1} and R^{A2} are methyl, R^{A3} and R^{A5} are methyl, and R^{A4} and R^{A6} are hydrogen, or
   R^{A1} and R^{A2} are methyl, and R^{A3} to R^{A6} are all hydrogen, or
   R^{A1} to R^{A6} are all hydrogen, or
   R^{A1} and R^{A2} are hydrogen, R^{A3} and R^{A5} are methyl, and R^{A4} and R^{A6} are hydrogen, or
   R^{A1} and R^{A2} are hydrogen, and R^{A3} to R^{A6} are methyl; 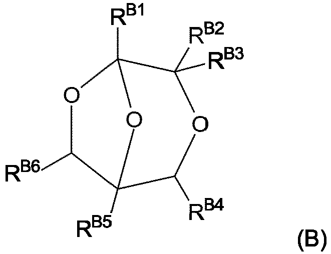
wherein R^{B1} and R^{B3} to R^{B6} are independently selected from hydrogen and methyl, and R^{B2} is selected from hydrogen, methyl, methoxy and ethoxy, and preferably:
   R^{B1} to R^{B6} are all hydrogen, or
   R^{B1} is methyl and R^{B2} to R^{B6} are hydrogen, or
   R^{B1} and R^{B2} are methyl and R^{B3} to R^{B6} are hydrogen, or
   R^{B1} to R^{B3} are methyl and R^{B4} to R^{B6} are hydrogen, or
   R^{B1} is hydrogen, R^{B2} is methyl and R^{B3} to R^{B6} are hydrogen, or
   R^{B1} to R^{B5} are hydrogen and R^{B6} is methyl, or
   R^{B1} and R^{B6} are methyl and R^{B2} to R^{B5} are hydrogen, or
   R^{B1} is hydrogen, R^{B2} is methyl, R^{B3} is methoxy and R^{B4} to R^{B6} are hydrogen, or
   R^{B1} is methyl, R^{B2} is methoxy and R^{B3} to R^{B6} are hydrogen, or
   R^{B1} is methyl, R^{B2} is ethoxy and R^{B3} to R^{B6} are hydrogen, or
   R^{B1} is methyl, R^{B2} is methoxy, R^{B3} is methyl and R^{B4} to R^{B6} are hydrogen, or
   R^{B1} is methyl, R^{B2} is ethoxy, R^{B3} is methyl and R^{B4} to R^{B6} are hydrogen, or
   R^{B1} is hydrogen, R^{B2} and R^{B3} are methyl and R^{B4} to R^{B6} are hydrogen; or
   R^{B1} is hydrogen, R^{B2} and R^{B3} are methyl, R^{B4} is hydrogen, R^{B5} is methyl and R^{B6} is hydrogen; 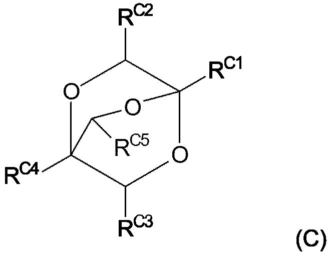
wherein R^{C1} to R^{C5} are independently selected from hydrogen and methyl, and preferably:
   R^{C1} to R^{C5} are all hydrogen, or
   R^{C1} is hydrogen, R^{C2} is methyl, and R^{C3} to R^{C5} are hydrogen, or
   R^{C1} and R^{C2} are hydrogen, R^{C3} is methyl, and R^{C4} and R^{C5} are hydrogen;
   R^{C1} is methyl and R^{C2} to R^{C5} are hydrogen, or
   R^{C1} and R^{C2} are methyl and R^{C3} to R^{C5} are hydrogen; 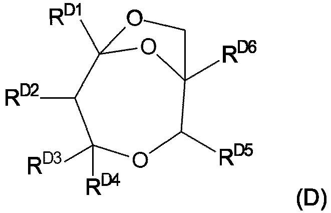
wherein R^{D1} is selected from hydrogen, methyl and ethyl and R^{D2} to R^{D6} are independently selected from hydrogen and methyl, and preferably:
   R^{D1} to R^{D6} are hydrogen, or
   R^{D1} is methyl, and R^{D2} to R^{D6} are hydrogen, or
   R^{D1} is ethyl and R^{D2} to R^{D6} are hydrogen, or
   R^{D1} and R^{D2} are methyl and R^{D3} to R^{D6} are hydrogen, or
   R^{D1} is methyl, R^{D2} to R^{D5} are hydrogen and R^{D6} is methyl, or
   R^{D1} is methyl, R^{D2} to R^{D4} are hydrogen, R^{D5} is methyl and R^{D6} is hydrogen, or
   R^{D1} is methyl, R^{D2} hydrogen, R^{D3} methyl, R^{D4} and R^{D5} hydrogen and R^{D6} is methyl, or
   R^{D1} is methyl, R^{D2} is hydrogen, R^{D3} and R^{D4} is methyl, R^{D5} is hydrogen and R^{D6} is methyl; 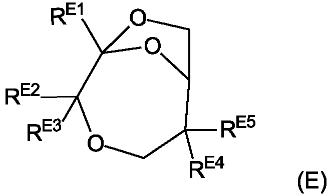
wherein R^{E1} to R^{E5} are independently selected from hydrogen and methyl, and preferably:
   R^{E1} to R^{E5} are all hydrogen, or
   R^{E1} is methyl and R^{E2} to R^{E5} are hydrogen, or
   R^{E1} to R^{E3} are hydrogen and R^{E4} and R^{E5} are methyl, or
   R^{E1} is methyl, R^{E2} and R^{E3} are hydrogen and R^{E4} and R^{E5} are methyl, or
   R^{E1} and R^{E2} are both methyl and R^{E3} to R^{E5} are hydrogen, or
   R^{E1} to R^{E3} are methyl and R^{E4} and R^{E5} are hydrogen, or
   R^{E1} and R^{E2} are methyl, R^{E3} is hydrogen and R^{E4} and R^{E5} are methyl, or
   R^{E1} to R^{E5} are all methyl;

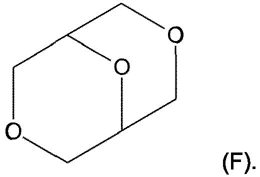

In line with the above, the following table summarizes the compounds from which the one or more types of a bridged, bicyclic compound are more preferably selected:

| Cpd. No. | Structure | Cpd. No. | Structure | Cpd. No. | Structure |
|---|---|---|---|---|---|
| 1 | 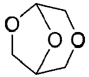 | 15 | 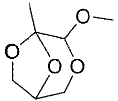 | 29 | 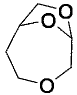 |
| 2 | 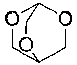 | 16 | 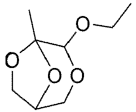 | 30 | 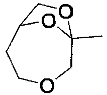 |
| 3 | 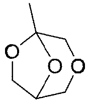 | 17 | 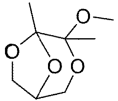 | 31 | 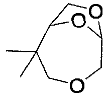 |
| 4 | 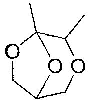 | 18 | 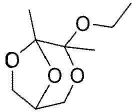 | 32 | 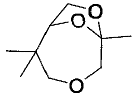 |
| 5 | 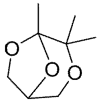 | 19 | 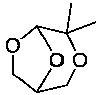 | 33 | 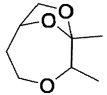 |
| 6 | 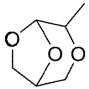 | 20 | 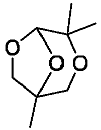 | 34 | 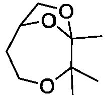 |
| 7 | 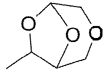 | 21 | 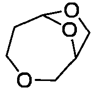 | 35 | 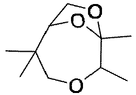 |
| 8 | 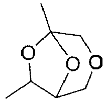 | 22 | 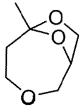 | 36 | 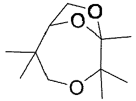 |
| 9 | 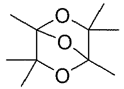 | 23 | 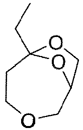 | 37 | 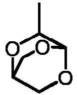 |
| 10 | 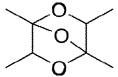 | 24 | 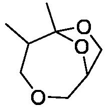 | 38 | 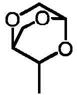 |
| 11 | 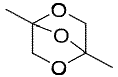 | 25 | 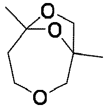 | 39 | 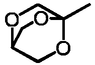 |
| 12 | 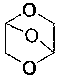 | 26 | 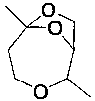 | 40 | 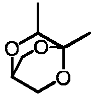 |
| 13 | 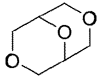 | 27 | 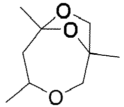 | 41 | 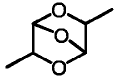 |
| 14 | 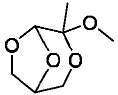 | 28 | 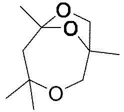 | 42 | 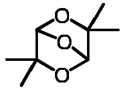 |

In view of the beneficial performance of the bridged, bicyclic compounds as solvents in solutions, a solvent as it is referred to in the context of the invention described herein is preferably a solvent which provides a solution. However, a use of one or more types of the bridged, bicyclic compounds as solvent(s) providing a dispersion, also referred to as dispersion solvents, is likewise contemplated as a part of the invention. As will be understood, this encompasses the case where the solvent provides a solution or a dispersion as a single solvent, or where it provides the solution or a dispersion as a component of a solvent formulation.

The solvent is further characterized in that it does not react with the substance(s) which are dissolved or dispersed in the solution or dispersion containing the solvent. Thus, if such a solvent is recovered from a solution or dispersion, the solvent molecules will generally retain their original structure which they had before the solution or dispersion was formed.

A solution as referred to herein, in line with the skilled person's understanding, represents a homogenous mixture, typically a liquid mixture, wherein a substance (also referred to as solute) is dissolved in a single solvent or in a solvent formulation such that the solvent or solvent formulation and the dissolved substance(s) form a single phase. It will be understood that, in a solution as it may be provided in the context of the first to fourth aspect of the invention, the single solvent would be represented by one type of the bridged, bicyclic compound selected from a trioxabicycloheptane, a trioxabicyclooctane, a trioxabicyclononane, and from alkylated and/or alkoxylated derivatives of any of these compounds as discussed herein, and the solvent formulation would comprise one or more types of the bridged, bicyclic compound selected from a trioxabicycloheptane, a trioxabicyclooctane, a trioxabicyclononane, and from alkylated and/or alkoxylated derivatives of any of these compounds as discussed herein as solvent(s), optionally in combination with a further solvent. Preferably, the dissolved substance(s) in such a solution is/are dissolved at a total concentration of 0.03 g or more, more preferably 0.05 g or more, per g of the solution.

A dispersion as referred to as referred in the various aspects of the invention represents a mixture with two or more phases, wherein a single solvent or a solvent formulation provides a continuous phase, typically a liquid continuous phase, which surrounds a substance that is present as a dispersed phase. As examples of such a dispersion, reference can be made to a suspension or an emulsion. It will be understood that, in a dispersion as it may be provided in the context of the first to fourth aspect of the present invention, the single solvent would be represented by one type of the bridged, bicyclic compound selected from a trioxabicycloheptane, a trioxabicyclooctane, a trioxabicyclononane, and from alkylated and/or alkoxylated derivatives of any of these compounds as discussed herein, and the solvent formulation would comprise one or more types of the bridged, bicyclic compound selected from a trioxabicycloheptane, a trioxabicyclooctane, a trioxabicyclononane, and from alkylated and/or alkoxylated derivatives of any of these compounds as discussed herein as solvent(s), optionally in combination with a further solvent. Preferably, the dispersed substance(s) in such a dispersion is/are dispersed at a total concentration of, 0.03 g or more, more preferably 0.05 g or more, per g of the solution.

A solvent formulation as referred in the context of the first to fourth aspect of the present invention is a medium, generally a liquid medium, which combines two or more components, including at least one type of the bridged, bicyclic compound selected from a trioxabicycloheptane, a trioxabicyclooctane, a trioxabicyclononane, and from alkylated and/or alkoxylated derivatives of any of these compounds as discussed herein. The components of the solvent formulation typically form a single-phase mixture. Preferably, the solvent formulation comprises one or more types of the bridged, bicyclic compound at a total concentration of 20 wt% or more, more preferably 50 wt% or more, based on the total weight of the solvent formulation.

As an example of a solvent formulation, reference can be made to a solvent mixture, i.e. a mixture of two or more solvents. A preferred example of such a solvent mixture is a solvent mixture which comprises or consists of two or more, preferably two, types of the bridged, bicyclic compound as solvents. As a further preferred example of a solvent formulation, reference can be made to a solvent mixture comprising or consisting of one or more types of the bridged, bicyclic compound as solvent(s) mixed with at least one further solvent which is not a bridged, bicyclic compound selected from a trioxabicycloheptane, a trioxabicyclooctane, a trioxabicyclononane, and from alkylated and/or alkoxylated derivatives of any of these compounds as discussed herein. In a solvent formulation which represents a solvent mixture, it is likewise preferred that the solvent mixture contains the one or more types of the bridged, bicyclic compound at a total concentration of 20 wt% or more, more preferably 50 wt% or more, based on the total weight of the solvent mixture. In order to more broadly exploit the benefits of the bridged, bicyclic compounds discussed therein, such a solvent mixture may also contain higher concentrations of the or more types of the bridged, bicyclic compound, e.g. a total concentration of 80 wt% or more, preferably 90 wt% or more, based on the total weight of the solvent mixture. As examples for further solvents useful as the at least one further solvent in a solvent mixture which is not a bridged, bicyclic compound selected from a trioxabicycloheptane, a trioxabicyclooctane, a trioxabicyclononane, and from alkylated and/or alkoxylated derivatives of any of these compounds as discussed herein, reference can be made to water, nitriles (preferably acetonitrile, propionitrile, or butyronitrile), esters (preferably ethyl acetate, ethyl-lactate (iso)propyl acetate, (iso)butyl acetate gamma-butyrolactone, or gamma-valerolactone), ethers (preferably 2-methyl tetrahydrofuran, tetrahydrofuran, 1,3-dioxolane, tetrahydropyran, isosorbide dimethyl ether, diethyl ether, dibutyl ether, triethylene glycol dimethyl ether, 1,4-dioxane , isopropyl ether, diethylene glycol dimethyl ether, tert-butyl methyl ether, ethylal, or 1,2-di(m)ethoxyethane), alcohols (preferably methanol, ethanol, (iso-)propanol, butanol, or ethylene glycol), hydrocarbons (preferably pentane, hexane, petroleum ether, toluene, xylene, or (methyl)cyclohexane), ketones (preferably acetone, or methyl (iso)butyl ketone), halogenated hydrocarbons (preferably dichloro(m)ethane, or chloroform) and to combinations thereof. A further solvent is preferably a solvent which is a liquid at room temperature.

As another example of a solvent formulation, reference can be made to a formulation comprising or consisting of a eutectic mixture of the one or more types of the bridged, bicyclic compound and at least one hydrogen bond donor compound. As examples of such hydrogen donor compounds, reference may be made to alcohols, preferably polyols containing two or more alcoholic hydroxy groups, carboxylic acids, carboxylic amides, ureas, thiourea, imidazole and imidaolinone. As preferred examples, reference may be made to phenol, resorcinol, catechol, propyl guaiacol, acetamide, oxalic acid, malonic acid, xylitol, urea, 1,1-dimethylurea, isosorbide, tartaric acid, tricarbalic acid, thiourea, trifluoracetamide, benzoic acid, itaconic acid, citric acid, imidazole, imidazolinone, benzamide, 4-hydroxybenzoic acid, cinnamic acid, ethylene glycol, propylene urea, phenylacetic acid, sorbitol, lactic acid, 1,3-dimethylurea, levulinic acid, gallic acid, caffeic acid, 1-methylurea, glycerol, succinic acid, hexanoic acid, coumaric acid, stearic acid, adipic acid, oleic acid and suberic acid. For example, the one or more types of the bridged, bicyclic compound and at least one hydrogen bond donor compound may be used in equimolar amounts in such an eutectic mixture (based on the total amount of the bridged, bicyclic compound(s)).

In the various aspects of the invention, the one or more types of the bridged, bicyclic compound are generally used as solvents in liquid form. It will be appreciated that this can be conveniently accomplished, e.g., by using them at a temperature which is at or above their melting point(s), and/or by selecting one or more bridged, bicyclic compounds having appropriate melting points for the intended application. As a further option, the use in a liquid solvent formulation is available. As will be appreciated by the skilled reader, the addition of a further solvent, as it may be contained in a solvent formulation in the form of a solvent mixture, can be relied on, where expedient, to provide a solvent formulation which is liquid at a temperature below the melting point of the bridged, bicyclic compound(s) contained therein. Likewise, a solvent formulation comprising or consisting of a eutectic mixture of the one or more types of the bridged, bicyclic compound and at least one hydrogen bond donor compound can be relied on to provide a solvent formulation which is liquid at a temperature below the melting point of the bridged, bicyclic compound(s) contained therein.

As noted above, a first aspect of the invention described herein relates to the use of one or more types of a bridged, bicyclic compound selected from a trioxabicycloheptane, a trioxabicyclooctane, a trioxabicyclononane, and from alkylated and/or alkoxylated derivatives of any of these compounds, as solvent(s). Preferred variants of the bridged, bicyclic compound are as discussed herein above, and particularly preferred as the bridged, bicyclic compound are one or more types selected from compounds 1 to 42 as disclosed herein.

As discussed above, the use of one or more types of the bridged, bicyclic compound as solvent(s) encompasses as options the use of one type of the bridged, bicyclic compound as a single solvent, or the use of one or more types of the bridged, bicyclic compound as solvent(s) comprised by a solvent formulation, preferably a solvent formulation comprising the bridged, bicyclic compound at total concentration of 20 wt% or more, more preferably at a total concentration of 50 wt% or more, based on the total weight of the solvent formulation. As also noted above, the bridged, bicyclic compounds are generally used as liquid single solvents or as solvent(s) comprised by a liquid solvent formulation.

Examples of useful solvent formulations are discussed above, and include in particular (i) solvent mixtures comprising or consisting of two or more, preferably two, types of the bridged, bicyclic compounds, (ii) solvent mixtures comprising or consisting of one or more types of the bridged, bicyclic compound mixed with at least one further solvent other than the bridged, bicyclic compounds as discussed herein, or (iii) eutectic mixtures of the one or more types of the bridged, bicyclic compound and at least one hydrogen bond donor compound.

In a general form, it is possible to use the one or more types of the bridged, bicyclic compound as solvent(s) which provide(s) a solution or a dispersion, preferably a solution, wherein a substance is dissolved or dispersed, preferably dissolved. The substance which can be dissolved or dispersed is not particularly limited, and a single substance, such as a single type of chemical compound, or mixtures of substances, such as a mixture of different types of chemical substances, can be used in this context.

As specific examples for the use of the one or more types of the bridged, bicyclic compound as solvent(s), reference can be made to the use as solvent(s) for a chemical reaction, the use as process solvent(s), the use as solvent(s) in polymer production and/or processing, the use as extraction solvent(s), the use as solvent(s) for purifying a substance, the use as solvent(s) for cleaning, the use as solvent(s) for the formulation and/or application of an active agent, or the use as solvent(s) for the storage of a substance.

When being used as solvent(s) for a chemical reaction, optionally as component of a solvent formulation, the one or more types of the bridged, bicyclic compound generally provide a reaction medium in the form of a solution or dispersion wherein at least one type of chemical reactant is dissolved or dispersed. The chemical reactant can thus be subjected to a chemical reaction in the solution or dispersion. To that extent, the invention also encompasses a method for carrying out a chemical reaction, which method comprises subjecting at least one type of chemical reactant to a chemical reaction in a solution or dispersion, which solution or dispersion contains one or more types of the bridged, bicyclic compound as a single solvent or as component of a solvent formulation.

It will be understood that the reference to the use as a solvent for a chemical reaction is not limited to a single reaction, but also encompasses cases where multiple reactions are carried out, e.g. in parallel or in sequence. This can be the case, e.g., in a polymerization process, or during peptide synthesis. It will be understood that the chemical reaction can be a reaction requiring only one type of reactant, e.g. an intramolecular reaction or a polymerization reaction, in which case only one type of reactant may be dissolved or dispersed. Alternatively, the chemical reaction can involve a reaction of two or more, such as two or three, reactants, in which case two or more types of reactants may be dissolved or dispersed.

The chemical reaction is preferably an organic synthesis reaction, such that the at least one type of chemical reactant preferably comprises at least one type of an organic compound.

It will be understood that the solution or dispersion can encompass further components which are known to the skilled person as being useful for carrying out the reaction, such as a catalyst or an initiator.

As examples for a chemical reaction, reference can be made to a cross-coupling reaction, an alkylation reaction, a hydrogenation reaction, an enzymatic reaction, an amidation reaction (amide bond formation reaction), an esterification reaction (ester bond formation reaction), an aromatic nucleophilic substitution, an acylation, a Grignard reagent formulation or a polymerization reaction. As specific examples, reference can be made to an aza-Michael addition, a Krapcho dealkoxycarbonylation, a Beckmann rearrangement, a Diels-Alder cycloaddition, a Menshutkin reaction, a Heck reaction or a Baylis-Hillman reaction.

In a preferred embodiment, the chemical reaction is peptide synthesis, such as liquid phase peptide synthesis or solid phase peptide synthesis, preferably solid phase peptide synthesis. In this case, the at least one type of chemical reactant comprises an amino acid or a derivative thereof carrying one or more protecting groups. As will be understood by the skilled reader, a reactant which is frequently used in peptide synthesis is an amino acid derivative wherein the amino group (or each amino group if more than one amino group is contained) is protected by a protecting group, such as Fmoc (fluorenylmethyloxycarbonyl) or Boc (tert-butyloxycarbonyl), preferably Fmoc. The carboxylic acid function of the amino acid or amino acid derivate is typically unprotected.

Thus, likewise preferred is the use of one or more types of a bridged, bicyclic compound selected from a trioxabicycloheptane, a trioxabicyclooctane, a trioxabicyclononane, and from alkylated and/or alkoxylated derivatives of any of these compounds, optionally as component of a solvent formulation, as solvent(s) for peptide synthesis such as liquid phase peptide synthesis or solid phase peptide synthesis, preferably solid phase peptide synthesis. When being used as solvent(s) for peptide synthesis, the one or more types of the bridged, bicyclic compound generally provide a reaction medium in the form of a solution wherein an amino acid or a derivative thereof carrying one or more protecting groups is dissolved. When used in solid phase peptide synthesis, the solution may be in contact with a solid phase carrier resin. When used in liquid phase peptide synthesis, the solution may contain a soluble tag to which an amino acid or a peptide can be bound. Also encompassed by the invention is a method for preparing a peptide, preferably via solid phase peptide synthesis, which method comprises subjecting at least one type of an amino acid or a derivative thereof carrying one or more protecting groups to an amide bond formation reaction in a solution which contains one or more types of the bridged, bicyclic compound as a single solvent or as component of a solvent formulation. When used in solid phase peptide synthesis, the solution may be in contact with a solid phase carrier resin. When used in liquid phase peptide synthesis, the solution may contain a soluble tag to which an amino acid or a peptide can be bound. It will be understood that the use as solvent for peptide synthesis and the method for preparing a peptide may also include a reaction in the solution, e.g. the solution in contact with the solid phase carrier or the solution containing a soluble tag, wherein an amino group is deprotected.

The increasing demand for therapeutic peptides and peptide-based drugs drives the urgent need for more sustainable synthetic methods. Solid-phase peptide synthesis (SPPS) is the method of choice to reliably synthesize peptides and small proteins. However, the current manufacturing processes compare unfavourably in terms of process mass intensity (PMI) to other modalities such as small molecules and biopharmaceuticals. The main contributor to the unfavourable PMI of SPPS is the excessive solvent usage. N,N-dimethylformamide (DMF), the most used solvent in SPPS, is classified as carcinogenic and reprotoxic. Consequently, the European Union has restricted DMF usage as of December 2023, highlighting the need for greener and safer alternatives. It has been found that the use of the bridged, bicyclic compounds as discussed herein as solvent(s) allows the peptide synthesis to be carried out with favorable results in terms of efficiency and product quality.

The use of one or more types of the bridged, bicyclic compounds discussed herein as solvent(s) for peptide synthesis, such as liquid phase peptide synthesis or solid phase peptide synthesis, preferably solid phase peptide synthesis, may in particular involve subjecting an amino acid or a derivative thereof carrying one or more protecting groups to an amide bond formation reaction in a solution which contains one or more types of the bridged, bicyclic compound as a single solvent or as component of a solvent formulation. For solid phase peptide synthesis, the amide bond formation may be carried out while the solution is in contact with a solid-phase carrier resin. The amide bond formation reaction can be carried out, for example, between the amino acid or the derivative thereof and a functional group, such as an amino group, provided on the solid-phase carrier resin. It can also be carried out between the amino acid or the derivative thereof and a functional group, such as an amino group, provided by an amino acid residue or a peptide residue which is already attached to the solid-phase carrier resin. For liquid phase peptide synthesis, the amide bond formation may be carried out in a solution containing a soluble tag to which an amino acid or a peptide can be bound. The amide bond formation reaction can in this case be carried out, for example, between the amino acid or the derivative thereof and a functional group, such as an amino group, provided on the soluble tag. It can also be carried out between the amino acid or the derivative thereof and a functional group, such as an amino group, provided by an amino acid residue or a peptide residue which is already attached to the soluble tag. Typically, the amide bond is formed between the C-terminus of an amino acid, generally an N-protected amino acid, and an amino group provided on the carrier resin or provided by an amino acid residue or a peptide residue which is already attached to the solid-phase carrier resin. As noted above, as a derivative of an amino acid carrying one or more protecting groups for use in peptide synthesis, such as liquid phase peptide synthesis or solid phase peptide synthesis, preferably solid phase peptide synthesis, a derivative is preferably used wherein the amino group (or each amino group if more than one amino group is contained) is protected by a protecting group, such as Fmoc (fluorenylmethyloxycarbonyl) or Boc (tert-butyloxycarbonyl), preferably Fmoc. A carboxylic acid function of the amino acid or amino acid derivate is typically unprotected. As noted above, the use as solvent for peptide synthesis may also include a reaction in the solution, e.g. the solution in contact with the solid phase carrier or the solution containing a soluble tag, wherein an amino group is deprotected.

When being used as process solvents, optionally as component of a solvent formulation, the one or more types of the bridged, bicyclic compound may, e.g., provide a solution or dispersion, preferably a solution, of one or more types of substances which allows the substances to be processed in liquid form, e.g. by pumping them or by mixing them.

When being used as solvents in polymer production, optionally as component of a solvent formulation, the one or more types of the bridged, bicyclic compound may, e.g., provide a solution or dispersion, preferably a solution, which allows one or more types of monomers to be dissolved or dispersed, preferably dissolved, therein to be subjected to a polymerization reaction. As exemplary polymers, reference can be made to a polyacrylate, a polymethacrylate (such as poly(methyl methacrylate)), a polyether, a polyurea, a polyacetal, a polyolefin, a polysulfone, a polyamide, a polyimide, a polyester (such as lactic acid or glycolic acid), polystyrene, polyacrylonitrile, polyvinylchloride, polyvinylpyrrolidone, polyvinylacetate, a polycarbonate, a polyurethane, an epoxy resin, a cellulose derivative, or a copolymer combining appropriate units of two or more of these exemplary polymers. To that extent, the invention also encompasses a method for preparing a polymer, which method comprises subjecting at least one type of monomer to a polymerization reaction in a solution or dispersion, which solution or dispersion contains one or more types of the bridged, bicyclic compound as a single solvent or as component of a solvent formulation.

When being used as solvents in polymer processing, optionally as component of a solvent formulation, the one or more types of the bridged, bicyclic compound may, e.g., provide a solution or dispersion, preferably a solution, wherein at least one type of polymer is dissolved or dispersed, preferably dissolved. As exemplary polymers, reference can be made to a polyacrylate, a polymethacrylate (such as poly(methyl methacrylate)), a polyether, a polyurea, a polyacetal, a polyolefin, a polysulfone, a polyamide, a polyimide, a polyester (such as lactic acid or glycolic acid), polystyrene, polyacrylonitrile, polyvinylchloride, polyvinylpyrrolidone, polyvinylacetate, a polycarbonate, a polyurethane, an epoxy resin, a cellulose derivative, or a copolymer combining appropriate units of two or more of these exemplary polymers. Such a solution or dispersion can be favorably used, e.g., to prepare a polymer blend. It can also be used to prepare a polymer film, a polymer coating, a polymer membrane, or a polymer fiber. As will be understood by the skilled reader, the preparation of such polymer products may be accomplished, e.g., by bringing the solution or dispersion of at least one type of polymer into the shape of a film, a coating, a membrane or a fiber, and removing the solvent or solvent formulation from the solution or dispersion to provide the film, coating, membrane or fiber. Also encompassed by the invention is a method for preparing a polymer film, a polymer coating, a polymer membrane or a polymer fiber, comprising as step of bringing a solution or dispersion of at least one type of polymer in a solution or dispersion, which solution or dispersion contains one or more types of the bridged, bicyclic compound as a single solvent or as component of a solvent formulation, into the form of a film, a coating, a membrane, or a fiber, and removing the solvent or the solvent formulation to provide the film, coating, membrane or fiber.

In a preferred embodiment, the one or more types of a bridged, bicyclic compound selected from a trioxabicycloheptane, a trioxabicyclooctane, a trioxabicyclononane, and from alkylated and/or alkoxylated derivatives of any of these compounds, is/are used, optionally as component of a solvent formulation, as solvent(s) in solution spinning, more preferably in a wet spinning process. As will be understood, in solution spinning, the one or more bridged, bicyclic compounds can be favorably used, optionally as component of a solvent formulation to provide the spinning solution wherein the polymer is dissolved from which the fiber is to be formed. In the wet spinning process, the spinning solution is typically extruded from a spinneret orifice into a coagulation bath in which the polymer is not soluble while the solvent or solvent formulation of the spinning solution are soluble, resulting in the solidification of the polymer in fiber form. As examples for suitable polymers, reference can be made to acrylonitrile or to polyamides. Also encompassed by the invention is a method for preparing a polymer fiber which comprises solution spinning of a at least one type of polymer in a solution or dispersion, which solution or dispersion contains one or more types of the bridged, bicyclic compound as a single solvent or as component of a solvent formulation.

When being used as extraction solvents, optionally as component of a solvent formulation, the one or more types of the bridged, bicyclic compound may, e.g., be contacted with a combination of substances to selectively dissolve (a) substance(s) contained in the combination of substances. Such a use is conceivable in diverse variations, e.g. for a liquid or a solid combination of substances, or for a combination of liquid and solid substances.

When being used as solvents for purifying a substance, optionally as component of a solvent formulation, the one or more types of the bridged, bicyclic compound may, e.g., be used to recrystallize a crystalline substance.

When being used as solvents for cleaning, optionally as component of a solvent formulation, the one or more types of the bridged, bicyclic compound may, e.g., be contacted with a substrate to be cleaned in order to dissolve or to disperse undesirable substances adhering to the substrate.

When being used as solvents for the formulation and/or the application of an active agent optionally as component of a solvent formulation, the one or more types of the bridged, bicyclic compound may, e.g., provide a solution or dispersion, preferably a solution, wherein at least one type of active agent is dissolved or dispersed, preferably dissolved. The provision of the active agent as a solution or dispersion may facilitate the mixing of the active agent with one or more auxiliary agents, and/or its application, e.g. by spraying a solution or dispersion onto a location to be treated by the active agent. As exemplary active agents, reference can be made to an agrochemical active agent, such as an insecticide, a herbicide, a fungicide, or a pesticide.

In line with the above, the one or more types of the bridged, bicyclic compound are preferably used as solvents, optionally as component of a solvent formulation, in a solution or dispersion wherein a substance is dissolved or dispersed which substance comprises at least one of the following (i) to (iv):
(i) at least one type of polymer, such as a polyacrylate, a polymethacrylate (such as poly(methyl methacrylate)), a polyether, a polyurea, a polyacetal, a polyolefin, a polysulfone, a polyamide, a polyimide, a polyester (such as lactic acid or glycolic acid), polystyrene, polyacrylonitrile, polyvinylchloride, polyvinylpyrrolidone, polyvinylacetate, a polycarbonate, a polyurethane, an epoxy resin, a cellulose derivative, or a copolymer combining appropriate units of two or more of these exemplary polymers;
(ii) at least one type of a monomer, such as a monomer suitable for the formation of an exemplary polymer or copolymer as referred to in (i);
(iii) at least one type of organic chemical reactant, such as an amino acid or a derivative thereof carrying one or more protecting groups, e.g. a protecting group for an amino group;
(iv) at least one type of an active agent, preferably an agrochemical active agent, such as an insecticide, a herbicide, a fungicide, or a pesticide.

A second aspect of the invention concerns a method for the preparation of a solution or dispersion, comprising a step of dissolving or dispersing a substance in a single solvent or in a solvent formulation, wherein the single solvent is a bridged, bicyclic compound selected from a trioxabicycloheptane, a trioxabicyclooctane, a trioxabicyclononane, and from alkylated and/or alkoxylated derivatives of any of these compounds and wherein the solvent formulation comprises one or more types of a bridged, bicyclic compound selected from a trioxabicycloheptane, a trioxabicyclooctane, a trioxabicyclononane, and from alkylated and/or alkoxylated derivatives of any of these compounds as solvent(s). Preferred variants of the bridged, bicyclic compound are as discussed herein above, and particularly preferred as the bridged, bicyclic compound are one or more types selected from compounds 1 to 42 as disclosed herein. The bridged, bicyclic compounds are generally used as liquid single solvents or as solvent(s) comprised by a liquid solvent formulation in order to dissolve or disperse the substance.

As will be understood from the above explanations, providing a solution is also a preferred embodiment in the context of this method, so that a preferred method is a method for the preparation of a solution, comprising a step of dissolving a substance in the single solvent or in the solvent formulation.

For the solvent formulation which may be used in the method of this aspect, it is likewise preferred that it comprises the one or more types of the bridged, bicyclic compound at a total concentration of 20 wt% or more, more preferably 50 wt% or more, based on the total weight of the solvent formulation. Examples of useful solvent formulations are discussed above, and include in particular (i) solvent mixtures comprising or consisting of two or more, preferably two, types of the bridged, bicyclic compounds, (ii) solvent mixtures comprising or consisting of one or more types of the bridged, bicyclic compound mixed with at least one further solvent other than the bridged, bicyclic compounds as discussed herein, or (iii) eutectic mixtures of the one or more types of the bridged, bicyclic compound and at least one hydrogen bond donor compound.

The substance to be dissolved or dispersed is not particularly limited, and a single substance, e.g. a single type of chemical compound, or two or more substances, such as a mixture of different types of chemical substances, can be used in this context. Since the method of this aspect is closely related to the first aspect of the invention, the information provided in the discussion of the first aspect of the invention about preferred substances which may be present in a solution or dispersion in the context of the invention also applies for the method of the second aspect.

As a consequence, the preparation of the solution or dispersion in accordance with the method of this second aspect may form part, e.g., of a process for carrying out a chemical reaction, a process for the production of a polymer, a process for the preparation of a polymer coating, a polymer film or a polymer membrane, a solution spinning process, a peptide synthesis, such as liquid phase peptide synthesis or solid phase peptide synthesis, preferably solid phase peptide synthesis, or a process for the formulation or the application of an active agent, e.g. an agrochemical agent.

For example, such a process for carrying out a chemical reaction may comprise the preparation of a solution or dispersion, comprising a step of dissolving or dispersing a substance in a single solvent or in a solvent formulation in accordance with the method defined hereinabove, wherein the substance comprises at least one type of a chemical reactant, followed by subjecting the chemical reactant to a chemical reaction in the solution or dispersion. Also in this context, the reference to a chemical reaction is not limited to a single reaction, but also encompasses cases where multiple reactions are carried out, e.g. in parallel or in sequence. The chemical reaction is preferably an organic synthesis reaction, such that the at least one type of chemical reactant preferably comprises at least one type of an organic compound.

Examples for a chemical reaction are the same as listed in the context of the use in accordance with the first aspect.

Thus, the method in accordance with the second aspect of the invention may be used in peptide synthesis, such as liquid phase peptide synthesis or solid phase peptide synthesis, preferably solid phase peptide synthesis. In accordance with a preferred embodiment, the method comprises a step of dissolving an amino acid or a derivative thereof carrying one or more protecting groups in the single solvent or in a solvent formulation as defined hereinabove. It is particularly preferred that an amino acid derivative is dissolved wherein the amino group (or each amino group if more than one amino group is contained) is protected by a protecting group, such as Fmoc (fluorenylmethyloxycarbonyl) or Boc (tert-butyloxycarbonyl), preferably Fmoc. The carboxylic acid function of the amino acid or amino acid derivate is typically unprotected.

In a related embodiment, a method for a synthesis of a peptide is provided, such as a method for liquid phase peptide synthesis or solid phase peptide synthesis, preferably solid phase peptide synthesis. The method comprises a step of dissolving or dispersing a substance in a single solvent or in a solvent formulation in accordance with the method defined hereinabove, wherein the substance comprises or consists of an amino acid or a derivative thereof carrying one or more protecting groups, followed by a step of carrying out an amide bond formation reaction. For solid phase peptide synthesis, the amide bond formation reaction may be carried out while the solution is in contact with a solid phase carrier resin. The amide bond formation reaction can be carried out, for example, between the amino acid or the derivative thereof and a functional group, such as an amino group, provided on the solid-phase carrier resin. It can also be carried out between the amino acid or the derivative thereof and a functional group, such as an amino group, provided by an amino acid residue or a peptide residue which is already attached to the solid-phase carrier resin. For liquid phase peptide synthesis, the amide bond formation may be carried out in a solution containing a soluble tag to which an amino acid or a peptide can be bound. The amide bond formation reaction can in this case be carried out, for example, between the amino acid or the derivative thereof and a functional group, such as an amino group, provided on the soluble tag. It can also be carried out between the amino acid or the derivative thereof and a functional group, such as an amino group, provided by an amino acid residue or a peptide residue which is already attached to the soluble tag. It will be understood that the method for preparing a peptide may also include a reaction in the solution, e.g. the solution in contact with the solid phase carrier or the solution containing a soluble tag, wherein an amino group is deprotected.

Also in line with the foregoing, provided is a method for the preparation of a polymer, which comprises a step of dissolving or dispersing a substance in a single solvent or in a solvent formulation in accordance with the method defined hereinabove, wherein the substance comprises at least one type of a monomer, followed by a step of subjecting the monomer to a polymerization reaction. Examples of polymers which can be prepared accordingly are the same as listed in the context of the use in accordance with the first aspect.

Likewise, provided are methods for the formation of a polymer film, a polymer membrane, a polymer coating and a polymer fiber, a step of dissolving or dispersing a substance in a single solvent or in a solvent formulation in accordance with the method defined hereinabove, wherein the substance comprises at least one polymer, followed by a step of bringing the solution or dispersion comprising at least one type of polymer into the shape of a film, a coating, a membrane or a fiber, and a step of removing the solvent or solvent formulation from the solution or dispersion to obtain the polymer film, the polymer coating, the polymer membrane or the polymer fiber.

In a further preferred embodiment a method is thus provided for preparing a polymer fiber by solution spinning, which method comprises a step of dissolving a substance in a single solvent or in a solvent formulation in accordance with the method defined hereinabove, wherein the substance comprises a polymer, to provide a spinning solution, followed by a step of extruding the solution from a spinneret orifice into a coagulation bath in which the polymer is not soluble while the single solvent or solvent formulation of the spinning solution is soluble, resulting in the solidification of the polymer in fiber form. As preferred examples for suitable polymers, reference can be made to acrylonitrile or to polyamides.

In line with the above, the method in accordance with the second aspect of the invention for the preparation of a solution or dispersion, which comprising a step of dissolving or dispersing a substance in a single solvent or in a solvent formulation as defined above, is preferably one wherein the dissolved or dispersed substance comprises at least one of the following (i) to (iv):
(i) at least one type of polymer, such as a polyacrylate, a polymethacrylate (such as poly(methyl methacrylate)), a polyether, a polyurea, a polyacetal, a polyolefin, a polysulfone, a polyamide, a polyimide, a polyester (such as lactic acid or glycolic acid), polystyrene, polyacrylonitrile, polyvinylchloride, polyvinylpyrrolidone, polyvinylacetate, a polycarbonate, a polyurethane, an epoxy resin, a cellulose derivative, or a copolymer combining appropriate units of two or more of these exemplary polymers;
(ii) at least one type of a monomer, such as a monomer suitable for the formation of an exemplary polymer or copolymer as referred to in (i);
(iii) at least one type of organic chemical reactant, such as an amino acid or a derivative thereof carrying one or more protecting groups, e.g. a protecting group for an amino group;
(iv) at least one type of an active agent, preferably an agrochemical active agent, such as an insecticide, a herbicide, a fungicide, or a pesticide.

According to a third aspect, a solution or dispersion is provided, said solution or dispersion comprising a substance which is dissolved or dispersed in a single solvent or in a solvent formulation, wherein the single solvent is a bridged, bicyclic compound selected from a trioxabicycloheptane, a trioxabicyclooctane, a trioxabicyclononane, and from alkylated and/or alkoxylated derivatives of any of these compounds and wherein the solvent formulation comprises one or more types of a bridged, bicyclic compound selected from a trioxabicycloheptane, a trioxabicyclooctane, a trioxabicyclononane, and from alkylated and/or alkoxylated derivatives of any of these compounds as solvent(s). Preferred variants of the bridged, bicyclic compound are as discussed herein above, and particularly preferred as the bridged, bicyclic compound are one or more types selected from compounds 1 to 42 as disclosed herein. As will be understood from the above explanations, the solution comprising a dissolved substance is a preferred embodiment of this aspect.

For the solvent formulation which may be used in the solution or dispersion of this aspect, it is likewise preferred that it comprises the one or more types of the bridged, bicyclic compound at a total concentration of 20 wt% or more, more preferably 50 wt% or more, based on the total weight of the solvent formulation. Examples of useful solvent formulations are discussed above, and include in particular (i) solvent mixtures comprising or consisting of two or more, preferably two, types of the bridged, bicyclic compounds, (ii) solvent mixtures comprising or consisting of one or more types of the bridged, bicyclic compound mixed with at least one further solvent other than the bridged, bicyclic compounds as discussed herein, or (iii) eutectic mixtures of the one or more types of the bridged, bicyclic compound and at least one hydrogen bond donor compound.

Moreover, in view of the close relationship between this aspect and the first and second relationship, the information provided in the context of these embodiments with regard to preferred dissolved or dispersed substances also applies in the context of this third embodiment. it is thus preferred that the substance which is dissolved or dispersed comprises at least one of the following (i) to (iv):
(i) at least one type of polymer, such as a polyacrylate, a polymethacrylate (such as poly(methyl methacrylate)), a polyether, a polyurea, a polyacetal, a polyolefin, a polysulfone, a polyamide, a polyimide, a polyester (such as lactic acid or glycolic acid), polystyrene, polyacrylonitrile, polyvinylchloride, polyvinylpyrrolidone, polyvinylacetate, a polycarbonate, a polyurethane, an epoxy resin, a cellulose derivative, or a copolymer combining appropriate units of two or more of these exemplary polymers;
(ii) at least one type of a monomer, such as a monomer suitable for the formation of an exemplary polymer or copolymer as referred to in (i);
(iii) at least one type of organic chemical reactant, such as an amino acid or a derivative thereof carrying one or more protecting groups, e.g. a protecting group for an amino group;
(iv) at least one type of an active agent, preferably an agrochemical active agent, such as an insecticide, a herbicide, a fungicide, or a pesticide.

A fourth aspect relates to a solvent formulation comprising comprising one or more types of a bridged, bicyclic compound selected from a trioxabicycloheptane, a trioxabicyclooctane, a trioxabicyclononane, and from alkylated and/or alkoxylated derivatives of any of these compounds at a total concentration of 20 wt% or more, preferably 50 wt% or more, based on the total weight of the solvent formulation, which solvent formulation is either
(i) a solvent mixture wherein the one or more types of a bridged, bicyclic compound is/are mixed with at least one further solvent which is not a bridged, bicyclic compound selected from a trioxabicycloheptane, a trioxabicyclooctane, a trioxabicyclononane, and from alkylated and/or alkoxylated derivatives of any of these compounds, or
(ii) a eutectic mixture wherein the one or more types of a bridged, bicyclic compound are mixed with at least one hydrogen bond donor compound.

Preferred variants of the bridged, bicyclic compound are as discussed herein above, and particularly preferred as the bridged, bicyclic compound are one or more types selected from compounds 1 to 42 as disclosed herein.

Also for this embodiment, the information provided above with regard to exemplary and preferred compositions of solvent formulations continues to apply. Thus, in the solvent formulation provided as a solvent mixture, the at least one further solvent is preferably selected from water, nitriles, esters, ethers, alcohols, hydrocarbons, ketones, halogenated hydrocarbons and from combinations thereof. Moreover, in the solvent mixture, the one or more types of the bridged, bicyclic compound are more preferably contained at a total concentration of 80 wt% or more, and still more preferably 90 wt% or more, based on the total weight of the solvent mixture.

Moreover, the solvent formulation which is a solvent mixture preferably contains the at least one further solvent at a concentration of 1wt% or more, based on the total weight of the solvent mixture.

### Methods of Preparation

As noted above, bridged, bicyclic compounds selected from a trioxabicycloheptane, a trioxabicyclooctane, a trioxabicyclononane, and from alkylated and/or alkoxylated derivatives as well as their synthesis are disclosed in the scientific literature, together with methods for their synthesis which can be relied on to provide these compounds. For example, reference can be made to the following documents:
- Gelas, Acétals cycliques dérivés de la glycérine. IV(*) - Isomérisation de dioxalannes et de dioxannes hydroxylés, Bulletin de la société chimique de france, 1970, nr 11, p4041-4046.
- Gelas, Acétals cycliques dérivés de la glycérine. V. Réactions de déchlorhydratation. Bulletin de la société chimique de france, 1970, nr 10, p.4046-4051.
- Gelas et al., Acétals cycliques dérivés de la glycérine; syntheses de trioxa-3,6,8 bicyclo[3.2.1]octanes et de trioxa,3,7,9 bicyclo[4.2.1]nonanes, Bulletin de la société chimique de france, 1970, nr 10, p.3721--3722.
- Calinaud et al., Recherches dans la série des acétals cycliques: XIV (*) - Synthèse de trioxa-2,5,7 bicyclo(2.2.1)heptanes. Bulletin de la société chimique de france, 1973, nr 9-10, p.2769-2773.
- Calinaud et al., Hétérocycles oxygénés. Synthèse d'alkyl-3,6,8-trioxabicyclo[3.2.1]octanes et d'alkyl-3,7,9 et -4,7,9-trioxabicyclo[4.2.1]nonanes. Can. J. Chem., 56, 2292 (1978).

In the context of the invention, an optimized method for the preparation of a trioxabicyclooctane or of an alkylated derivative thereof is additionally provided, which
comprises:
a) a step wherein a compound of formula (S1) 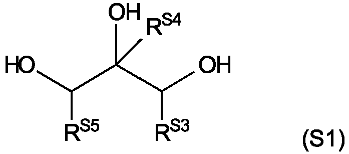 wherein R^{S3}, R^{S4} and R^{S5} are independently hydrogen or methyl, and are preferably all hydrogen,
   is reacted with a compound of formula (S2A) or a compound of formula (S2B) 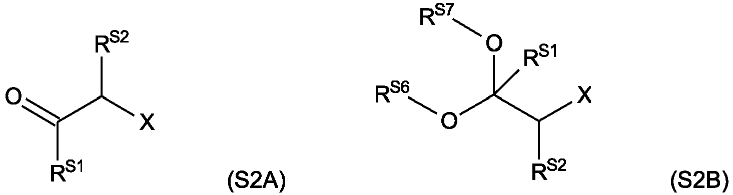 wherein
      X is Cl or Br, and is preferably Cl,
      R^{S1} and R^{S2} are independently hydrogen or methyl, preferably R^{S1} and R^{S2} are both hydrogen or R^{S1} is methyl and R^{S2} is hydrogen,
      R^{S6} and R^{S7} are C1-C3 alkyl, and are preferably both methyl;
   to obtain a mixture of isomer compounds (S3T), (S3C), (S4T) and (S4C), wherein X, R^{S1}, R^{S2}, R^{S3}, R^{S4} and R^{S5} are defined as above, 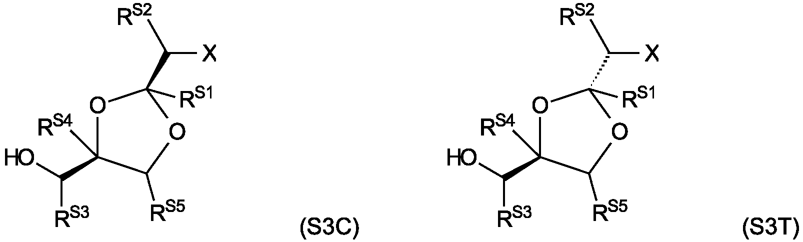 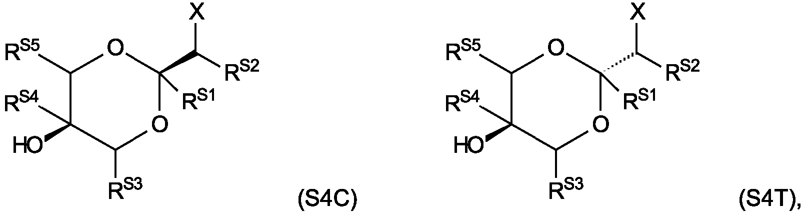 and
b) a step wherein the compounds of formula (S3C) and (S4C) are subjected to intramolecular ether in the presence of NaOH or KOH, preferably NaOH, in an amount of 1 to 2.70 molar equivalents, relative to the total amount of compounds of formula (S3C) and (S4C), in water,
   to obtain the compounds of formula (S5) and (S6), wherein R^{S1}, R^{S2}, R^{S3}, R^{S4} and R^{S5} are as defined above:

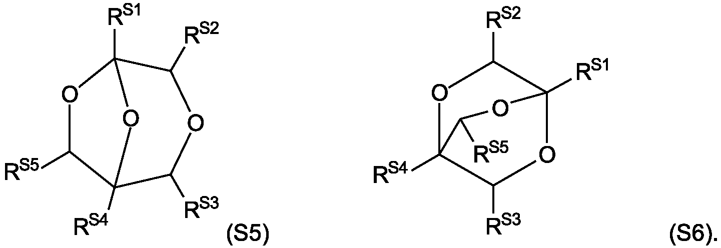

Preferably, in the above formulae, X is Cl and R^{S1} to R^{S5} are hydrogen. It has been found that, by using NaOH in water as a base in step b) in the indicated equivalent amounts, the cis isomers (S3C) and (S4C) can be efficiently converted to TBO products while the trans isomers (S3T) and (S4T) are preserved (low trans-isomer conversion).

As opposed to the use of strong organic bases such as t-BuOK in organic media, the use of moderate equivalent amounts of NaOH (or KOH) in aqueous solutions in step b) allows for high yields of the TBO, while greatly preserving the non-ring closing trans-isomers (*trans-*isomer conversion < 30 %) when keeping NaOH equivalents below 2.70 compared to *cis-*isomers). This allows for further valorization of the trans-isomers in other applications.

### General Remarks

As will be understood, the use of "a" or "an" as indefinite article includes herein, unless indicated otherwise or inappropriate in a specific context, encompasses the singular and the plural of the following noun. The use of "or", unless indicated otherwise, or inappropriate in a given context, does not indicate that the options linked by the "or" are mutually exclusive. Where expedient, this is further underlined by the use of "and/or", which likewise indicates that the options linked accordingly can be selected in any combination, or singly, such that, e.g., A and/or B means that A can be selected in the absence of B, B can be selected in the absence of A, and A and B can be selected together.

In this specification, a number of documents are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

### Examples

### 1. Synthesis

### Preparation of a mixture of TBO compounds 1 and 2

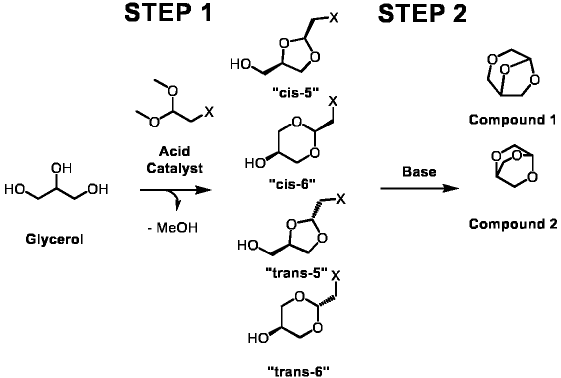

*Reaction scheme for TBO synthesis via Williamson etherification of halogenated acetals*

### Example STEP 1 (X = chlorine):

Glycerol (50.0 g, 0.537 mol, 1.0 eq) and chloro acetaldehyde dimethyl acetal (68.3 g, 0.537 mol, 1.0 eq) were added in a 250 mL round bottom flask. Then, Amberlyst-15 (1.15 g) was added. The flask was connected to a distillation bridge, connected to a cold methanol trap, cooled in an ice bath. The mixture was heated at 100 °C for 4 hours, under stirring, under reduced pressure (800 mbar) to remove produced MeOH. The resulting solution was cooled to room temperature, the Amberlyst-15 catalyst filtered off, and the resulting mixture analysed with Gas-chromatography-FID:
Yield **("cis"2-(chloromethyl)-1,3-dioxan-5-ol)** = **"6-cis":** 13.36 %
Yield **("trans" 2-(chloromethyl)-1,3-dioxan-5-ol)** = **"6-trans"** 10.31 %
Yield **("*cis*" 2-chloromethyl-4-hydroxymethyl-1,3-dioxolane)** = **"5-cis":** 30.63 %
Yield **("trans"2-chloromethyl-4-hydroxymethyl-1,3-dioxolane):** = **"5 -trans":** 26.33 %

The four isomers can be purified via vacuum distillation, and/or separated via Flash Column Chromatography, and individually identified by Nuclear Magnetic Spectroscopy (NMR):
***"cis"* 2-(chloromethyl)-1,3-dioxan-5-ol** ¹H NMR (CDCl₃): *δ* 4.73 (t, 1 H), 4.05 (m 2 H), 3.91 (m, 2H), 3.53 (m, 1 H), 3.51 (m, 2 H).
***"trans"* 2-(chloromethyl)-1,3-dioxan-5-ol** ¹H NMR (CDCl₃): *δ* 4.62 (t, 1 H), 4.21 (m, 2 H), 3.91 (m, 1 H), 3.51 (d, 2 H), 3.42 (m, 2 H).
**"*cis*" 2-chloromethyl-4-hydroxymethyl-1,3-dioxolane** ¹H NMR (CDCl₃): *δ* 5.09 (t, 1 H), 4.19 (m, 1 H), 3.93 (t, 1 H), 3.82 (t, 1 H), 3.57 (d, 2 H), 3.46 (d, 2 H).
***"trans"* 2-chloromethyl-4-hydroxymethyl-1,3-dioxolane ¹**H NMR (CDCl₃): *δ* 5.21 (t, 1 H), 4.24 (m, 1 H), 4.08 (t, 1H), 3.71 (t, 1 H), 3.65 (m, 2 H), 3.55 (d, 2 H).

### Example STEP 2 (X = chlorine):

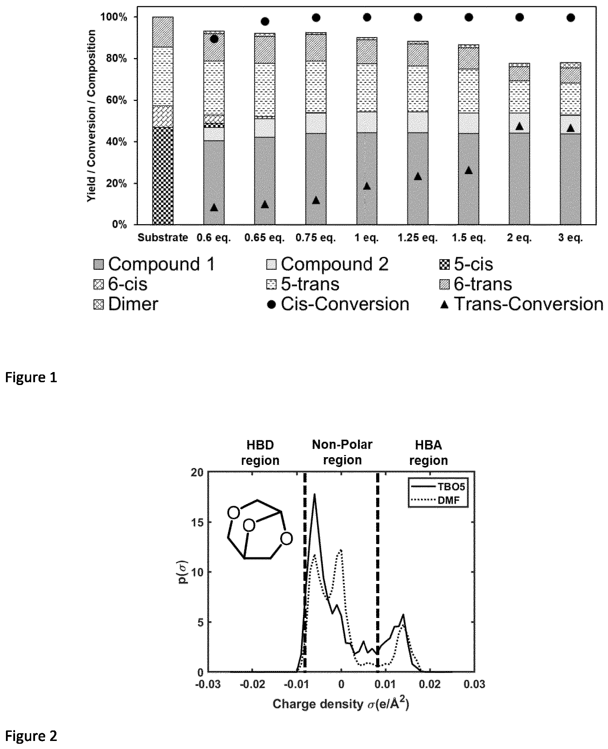

A distilled mixture of chloro-acetals (2g, composition as indicated by "Substrate" entry in Figure 1: **"5-cis"** 46.5 mol%, **"6-cis"** 10.3 mol%, **"5-trans"** 27.9 mol%, **"6-trans"** 14.3 mol%) was added to a 10 mL round bottom crimp cap vial, together with a aqueous NaOH solution (2 mL, molar equivalents of NaOH compared to all 4 isomers as indicated in Figure 1). Under stirring, the vials were heated at 100 °C for 5 hours. The resulting solution was cooled to room temperature, water was added to a total volume of 4 mL, and the reaction mixture was extracted five times with 3 mL of ethyl acetate. The organic phase was collected and analysed with Gas-Chromatography-FID.

Figure 1 illustrates the optimization of eq. of NaOH during Williamson etherification for compounds 1+2 synthesis.

Compound 1 and 2 can be purified as a mixture via vacuum distillation, and/or separated via Flash Column Chromatography, and individually identified by Nuclear Magnetic Spectroscopy (NMR):
**Compound 1:**¹H NMR (CDCl₃): *δ* 5.27 (m, 1 H), 4.35 (m, 1 H), 4.21 (m, 1 H), 3.88 (m, 1 H), 3.78 (m, 1H), 3.57 (m, 2 H), 3.48 (m, 1H). ¹³C NMR (CDCl₃): *δ* 98.8, 72.4, 68.6, 66.1, 63.9.
**Compound 2** ¹H NMR (CDCl₃): *δ* 4.82 (m, 1 H), 4.31 (m, 2 H), 4.18 (m, 2 H), 4.09 (m, 2 H), 3.77 (m, 1H). ¹³C NMR (CDCl₃): *δ* 87.8, 70.3, 68.6, 68.6, 66.1.

As visible in Figure 1 for the range 0.6 eq-1.5 molar equivalents NaOH, (or 1 to 2.63 equivalents compared to only the cis-isomers), cis-isomers can be converted nearly quantitively towards the target compounds 1 and 2.

### 2. Physical and solvation properties of TBH, TBO, TBN

### General physical properties

**Table 1**

| | Structure | Melting point (°C) | Boiling point (°C) | Solubility in water | Density (g/mL) |
|---|---|---|---|---|---|
| 1 | 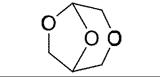 | 54 °C^{a} | 70 (at 15 mmHg) ^{a} | ∞ | 1.21 ^{c} |
| | | | 185 (at 760 mmHg) ^{a,b} | | |
| 2 | 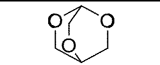 | | | | |
| 3 | 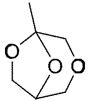 | n/m (< 20 °C) | 61 (at 13 mmHg) ^{d} | ∞ | n/m |
| | | | 176 (at 760 mmHg) ^{b} | | |
| 4 | 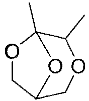 | n/m (< 20 °C) | 68 (at 15 mmHg) | n/m | n/m |
| | | | 181 (at 760 mmHg) ^{b} | | |
| 5 | 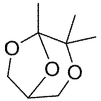 | 51 °C^{d} | 71 (at 12 mmHg) ^{e} | n/m | n/m |
| | | | 190 (at 760 mmHg) ^{b} | | |
| 6 | 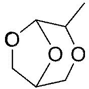 | n/m | 80 (at 25 mmHg) ^{f} | n/m | n/m |
| | | | 183 (at 760 mmHg) ^{b} | | |
| 7 | 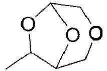 | n/m | n/m | n/m | n/m |
| 8 | 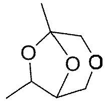 | n/m | n/m | n/m | n/m |
| 9 | 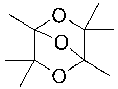 | n/m (< 20 °C) | 63 (at 19 mmHg) ^{g} | n/m | n/m |
| | | | 170 (at 760 mmHg) ^{b} | | |
| 10 | 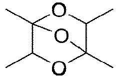 | n/m (<20 °C) | 42 (at 10 mmHg) ^{g} | n/m | n/m |
| | | | 158 (at 760 mmHg) ^{b} | | |
| 11 | 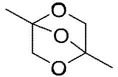 | n/m | n/m | n/m | n/m |
| 12 | 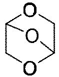 | n/m | n/m | n/m | n/m |
| 13 | 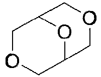 | 122 ^{h} | 209 ^{h} | n/m | n/m |
| 14 | 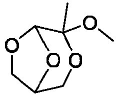 | n/m | 88 (at 10 mmHg) ⁱ | n/m | n/m |
| | | | 214 (at 760 mmHg) ^{b} | | |
| 15 | 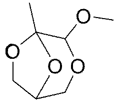 | n/m | 87 (at 10 mmHg) ⁱ | n/m | n/m |
| | | | 213 (at 760 mmHg) ^{b} | | |
| 16 | 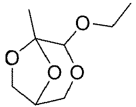 | n/m | 89 (at 10 mmHg) ⁱ | n/m | n/m |
| | | | 215 (at 760 mmHg) ^{b} | | |
| 17 | 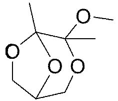 | n/m | 78 (at 4 mmHg) ⁱ | n/m | n/m |
| | | | 223 (at 760 mmHg) ^{b} | | |
| 18 | 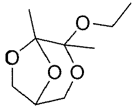 | n/m | 82 (at 4 mmHg)ⁱ | n/m | n/m |
| | | | 228 (at 760 mmHg) ^{b} | | |
| 19 | 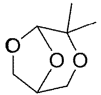 | n/m | 74 (at 11 mmHg) ^{j} | n/m | n/m |
| | | | 195 (at 760 mmHg) ^{b} | | |
| 20 | 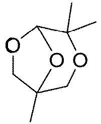 | n/m | 66 (at 11 mmHg) ^{j} | n/m | n/m |
| | | | 185 (at 760 mmHg) ^{b} | | |
| 21 | 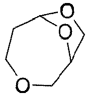 | n/m | 80 (at 12 mmHg) ^{k} | n/m | n/m |
| | | | 201 (at 760 mmHg) ^{b} | | |
| 22 | 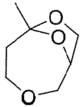 | n/m | 75 (at 12 mmHg) ^{k} | n/m | n/m |
| | | | 195 (at 760 mmHg) ^{b} | | |
| 23 | 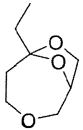 | n/m | 87 (at 12 mmHg) ^{k} | n/m | n/m |
| | | | 210 (at 760 mmHg) ^{b} | | |
| 24 | 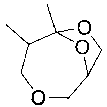 | n/m | 80 (at 12 mmHg) ^{k} | n/m | n/m |
| | | | 201 (at 760 mmHg) ^{b} | | |
| 29^{l} | 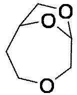 | n/m | n/m | n/m | n/m |
| n/m: Not measured | | | | | |
| ^{a} Mixture of compound 1 (81.2 mol%) and 2 (17.1 mol%) | | | | | |
| ^{b} Extrapolated to atmospheric pressure with Sigma Aldrich's Nomograph tool | | | | | |
| ^{c} Mixture of compound 1 (74.9 mol%) and 2 (24.8 mol%) at 60 °C | | | | | |
| ^{d} Value from J. Gelas, R. Rambaud, Bull. Soc. Chim, 6, (1970), 2386-2392 | | | | | |
| ^{e} Value from J. Gelas, Bull. Soc. Chim, 10, (1970), 3721-3722 | | | | | |
| ^{f} Value from Piergentili et al. Bioorganic & Medicinal Chemistry 17 (2009) 8174-8185 | | | | | |
| ^{g} Value from P. Calinaud et al., Bull. Soc. Chim, 9-10, (1973), 2769-2773 | | | | | |
| ^{h} Value from Summerbell et al., J Am Oil Chem Soc 39 (1962), 306; Stoehr, J. Prakt. Chemie, 2, 55 (1897), 78. | | | | | |
| ⁱ Value from Gelas et al. Carbohydrate research, 30 (1973) 21-34 | | | | | |
| ^{j} Value from P. Calinaud et al. Bull. Soc. Chim, 5-6, (1974), 1155-1157 | | | | | |
| ^{k} Value from J. Gelas. Bull. Soc. Chim, 11, (1970), 4041-4046 | | | | | |
| ^{l} P. Calinaud et al. Canadian Journal of Chemistry. Vol. 56; 17. P. 2292-2300. | | | | | |

### Flash points

In terms of safety, the greatest risk of injury is coming from solvents with a lower flash point, e.g., 2-MeTHF (-10 °C), CPME (-1 °C), 1,4-dioxane (12 °C), DMF (58 °C).

Flash point of a TBO (mixture of compound 1, 69 mol%, and compound 2, 28 mol%) was measured to be 71 °C (measured in duplicate, closed-cup). In this regard, TBO (mixture compound 1 and 2) , is a much more advantageous alternative.

### Miscibility of TBO compound (1 + 2) with common organic solvents

The miscibility of TBO (compound 1+ 2) with other organic solvents was determined by heating a glass vial containing a known volume of TBO in a heating block at 70°C. Then, equal volume of a given investigated organic solvent was added to the vial and the mixture was vortexed for one minute. The miscibility of TBO with investigated solvents was determined after cooling down.

| **Solvent** | **Miscibility with TBO compound 1+2** |
|---|---|
| Acetone | Miscible |
| Acetonitrile | Miscible |
| Chloroform | Miscible |
| CPME | Miscible |
| Cyclohexane | Non-miscible |
| DMF | Miscible |
| DMSO | Miscible |
| 1,4-Dioxane | Miscible |
| Di-*n*-butyl ether | Non-miscible* |
| Ethanol | Miscible |
| Ethyl acetate | Miscible |
| Hexane | Non-miscible |
| Isopropanol | Miscible |
| 2-MeTHF | Miscible |
| Toluene | Miscible |

| | |
|---|---|
| * Although miscible at 70°C, the mixture of TBO and di-n-butyl ether phase separated upon cooling down, followed by the solidification of the TBO layer. The other solvent mixtures appeared liquid and homogeneous upon cooling. | |

### Stability of TBO

The chemical stability of TBO (Compound 1+2 mixture) was determined by placing a sample of TBO (compound 1+2) (2 g) in a vial at 60°C under stiring along with a base (CsCO₃, KOAc, K₂CO₃, NaOH, pyridine; 1 mmol). Color change was visually assessed and TBO relative concentration was quantified by GC-FID after 1, 24, and 48 hours. No GC-FID quantification were performed for samples that solidified upon unequivocal degradation.

| Base | Aspect change | | | | Degradation | | | | | |
|---|---|---|---|---|---|---|---|---|---|---|
| | 1 h | 24 h | 48 h | | 1 h | | 24 h | | 48 h | |
| CsCO₃ KOAc | No | No | | No No No | No | No | No | No | No | No |
| K₂CO₃ NaOH | No | No | | Yellowish | No | No | No | No | No | No |
| Pyridine | No | No | | Yellowish | No | | No | | No | |
| | Yellowish | | Yellowish | | | | | | | |
| | Yellowish | | Yellowish | | | | | | | |

For TBO (Compound 1+2 mixture), it is shown that the TBO structure is very stable towards (strong) bases. This is a great advantage in comparison to other traditional solvents.

### Modelling of solvent properties: COSMO RS

Having established the main physical properties of listed TBH/TBO/TBN, numerical simulations were performed to assess the structures' chemical properties and potential as solvent. In that regard, COSMO-RS modeling was first performed. Conductor-like screening model for real solvents (COSMO-RS) is a computational method based on quantum mechanics that allows to compute surface-charge density via dielectric screening. The results of these calculations are used to construct a map of surface- charge density around the molecule, called sigma surface, and a corresponding surface-charge density distribution, called sigma profile.

COSMO-RS data were obtained with SCM's AMSjobs suit. TBH, TBO and TBN molecular structures was input, geometry was pre-optimized with a universal force field, then a COSMO file was generated using the "Solvent CRS" task, with COSMO solvation method, GGA:BP density functional, triple-zeta basis set (TZP) and otherwise default parameters. Thus generated COSMO files were used to generate associated COSMO surface and sigma profile using the COSMO-RS software of the AMSjobs suit. Sigma profiles of common solvents were generated using the built-in library of COSMO-data.

As shown in Figure 2, sigma profiles can be qualitatively divided into three main regions: hydrogen bond donor region hydrogen bond donor region (*σ* < -0.0082 e/Å²), hydrogen bond acceptor region (*σ* > +0.0082 e/Å²), and a non-polar region (-0.0082 < *σ* < +0.0082 e/Å²).

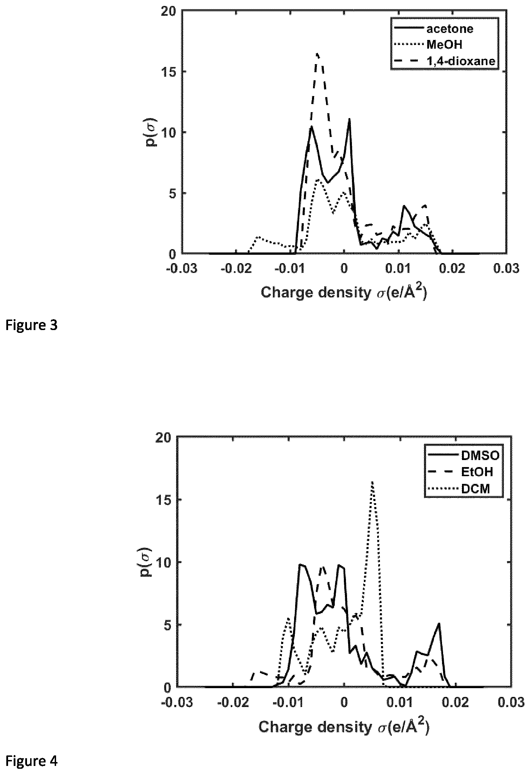

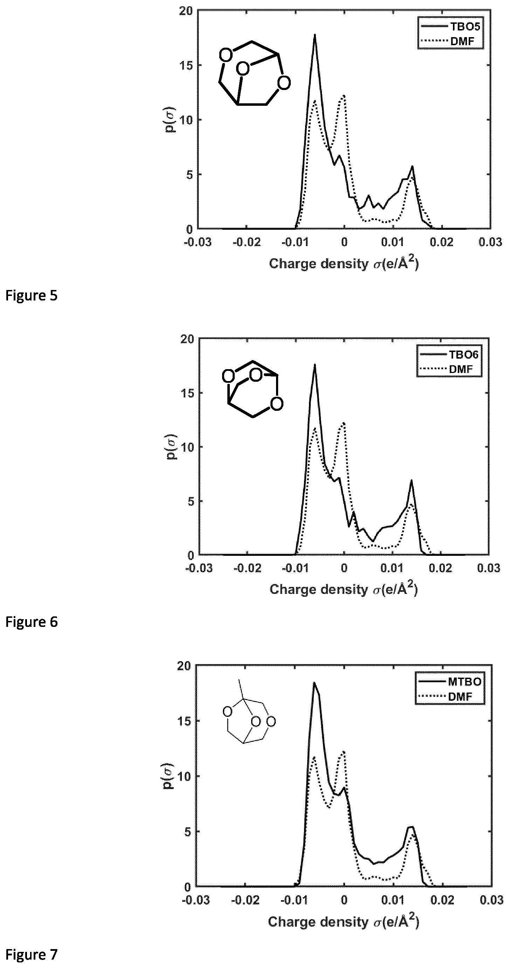

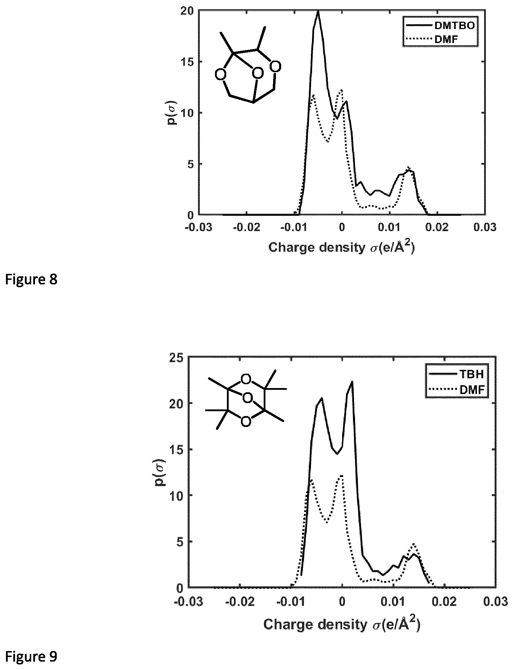

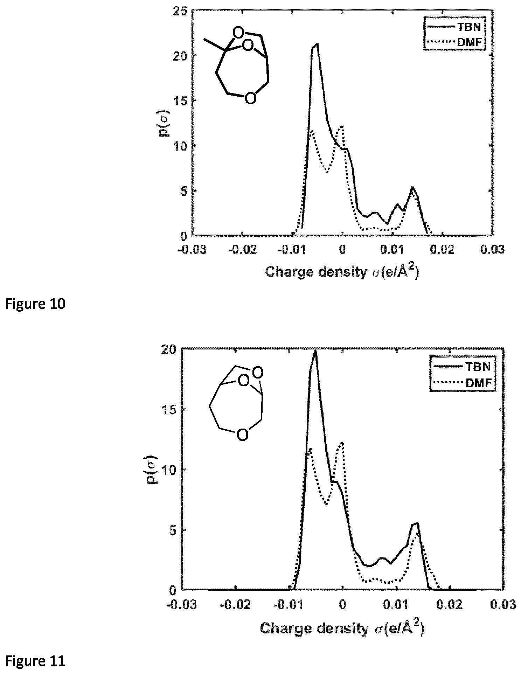

Thus, as represented by Figures 3 to 11, all TBH/TBO/TBN examples have one of the sigma profile peaks located in the non-polar region, and the other one is located in the hydrogen bond acceptor region. This is consistent with TBH/TBO/TBN's oxygenated chemical structures; oxygens are good hydrogen bond acceptors. Similarly, the absence of peak in the hydrogen bond donor region is consistent with TBH/TBO/TBN's aprotic nature. Furthermore, TBH/TBO/TBNs appear to exhibit sigma profile of similar shape as traditional dipolar aprotic solvents (e.g. DMF, DMSO) and ethereal (e.g. 1,4-dioxane) which is a good starting point in view of the replacement of these. Inversely, it can be seen that TBH/TBO/TBN differ from protic solvents (e.g. MeOH) by the absence of a hydrogen bond donor peak, and from rather apolar solvents (e.g. DCM) by the presence of a hydrogen bond acceptor peak.

Figure 3: COSMO-RS Sigma profiles of traditional solvents.

Figure 4: COSMO-RS Sigma profiles of traditional solvents (part 2).

Figure 5. COSMO-RS Sigma profiles of COMPOUND 1 compared to traditional DMF.

Figure 6: COSMO-RS Sigma profiles of COMPOUND 2 compared to traditional DMF.

Figure 7: COSMO-RS Sigma profiles of COMPOUND 3 compared to traditional DMF.

Figure 8: COSMO-RS Sigma profiles of COMPOUND 4 compared to traditional DMF

Figure 9 COSMO-RS Sigma profiles of COMPOUND 9 compared to traditional DMF

Figure 10: COSMO-RS Sigma profiles of COMPOUND 22 compared to traditional DMF

Figure 11. COSMO-RS Sigma profiles of COMPOUND 29 compared to traditional DMF

### Modelling of Solvent Properties: Hansen Solubillity Parameters

Modelling of solvent properties: Each solvent can be characterized by three Hansen parameters, each generally measured in MPa^{0.5}.

δD: The energy from dispersion forces between molecules

δP: The energy from dipolar intermolecular force between molecules

δH: The energy from hydrogen bonds between molecules

All three parameters were calculated for compounds 1 to 42 using HSPiP 5.3.02 Software (compound 1 and 2), or 6.0.04 (for the other compounds).

**Table 2: Hanssen Solubility Parameters of the solvent compounds**

| | **Structure** | **HSP** | | | **Closest Match Solvent** |
|---|---|---|---|---|---|
| | | **6D** | **δP** | **δH** | |
| 1 | 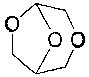 | 17.9 | 8.6 | 7.8 | Dimethyl isosorbide |
| 2 | 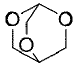 | 17.9 | 8.6 | 7.8 | Dimethyl isosorbide |
| 3 | 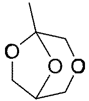 | 17.8 | 8.1 | 6.6 | Dimethyl isosorbide |
| 4 | 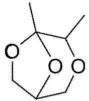 | 17.3 | 6.7 | 5.5 | Isophorone |
| 5 | 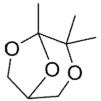 | 16.8 | 5.6 | 4.4 | Tributyl phosphate |
| 6 | 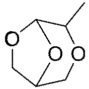 | 17.1 | 7.0 | 6.2 | Methylene Chloride |
| 7 | 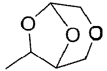 | 17.1 | 7.0 | 6.2 | Isophorone |
| 8 | 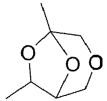 | 17.3 | 6.7 | 5.5 | Isophorone |
| 9 | 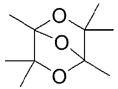 | 16.2 | 4 | 2.6 | Di-Isobutyl Ketone |
| 10 | 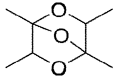 | 17.0 | 5.5 | 4.2 | Cyclopentyl Methyl Ether |
| 11 | 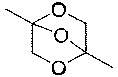 | 17.9 | 8.2 | 6.2 | Cyclohexanone |
| 12 | 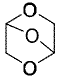 | 17.5 | 9.0 | 7.6 | Dimethyl isosorbide |
| 13 | 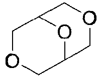 | 17.7 | 8.0 | 6.9 | Dimethyl isosorbide |
| 14 | 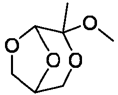 | 17.3 | 7.8 | 6.2 | Methylene Chloride |
| 15 | 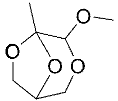 | 17.3 | 7.8 | 6.2 | Methylene Chloride |
| 16 | 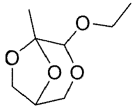 | 17.1 | 7.2 | 5.6 | Isophorone |
| 17 | 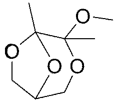 | 17.5 | 7.3 | 5.5 | Cyclohexanone |
| 18 | 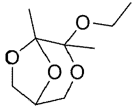 | 17.3 | 6.8 | 5.0 | Isophorone |
| 19 | 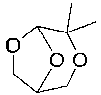 | 16.6 | 5.8 | 5 | Tributyl Phosphate |
| 20 | 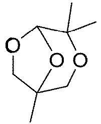 | 16.8 | 5.6 | 4.4 | Tributyl Phosphate |
| 21 | 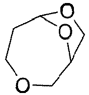 | 17.6 | 7.6 | 7.1 | Dimethyl isosorbide |
| 22 | 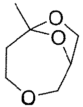 | 17.8 | 7.3 | 6.3 | Dimethyl isosorbide |
| 23 | 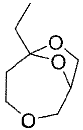 | 17.5 | 6.7 | 5.7 | Isophorone |
| 24 | 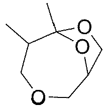 | 17.5 | 6.3 | 5.2 | Isophorone |
| 25 | 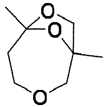 | 17.9 | 6.9 | 5.6 | Butyl Benzoate |
| 26 | 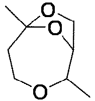 | 17.4 | 6.1 | 5.4 | Butyl Benzoate |
| 27 | 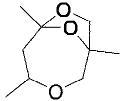 | 17.6 | 6.0 | 4.6 | Butyl Benzoate |
| 28 | 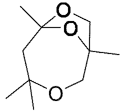 | 17.1 | 5.2 | 3.7 | Cyclopentyl methyl ether |
| 29 | 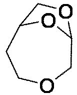 | 17.6 | 7.6 | 7.1 | Dimethyl isosorbide |
| 30 | 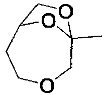 | 17.8 | 7.3 | 6.3 | Dimethyl isosorbide |
| 31 | 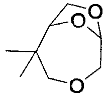 | 16.8 | 5.6 | 4.8 | Tributyl Phosphate |
| 32 | 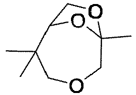 | 17.0 | 5.4 | 4.2 | Cyclopentyl methyl ether |
| 33 | 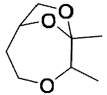 | 17.4 | 6.1 | 5.4 | Butyl benzoate |
| 34 | 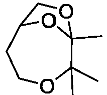 | 16.9 | 5.2 | 4.4 | Cyclopentyl methyl ether |
| 35 | 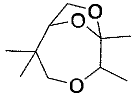 | 16.7 | 4.6 | 3.6 | Cyclopentyl methyl ether |
| 36 | 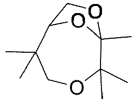 | 16.4 | 3.9 | 2.9 | Di-Isobutyl Ketone |
| 37 | 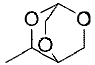 | 17.1 | 7.0 | 6.2 | Isophorone |
| 38 | 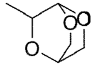 | 17.1 | 7.0 | 3.2 | Isophorone |
| 39 | 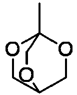 | 17.8 | 8.1 | 6.6 | Dimethyl isosorbide |
| 40 | 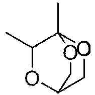 | 17.3 | 6.7 | 5.5 | Isophorone |
| 41 | 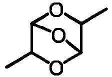 | 16.5 | 5.9 | 5.4 | Tributyl Phosphate |
| 42 | 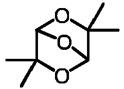 | 15.6 | 4.1 | 3.4 | Di-Isobutyl Ketone |
| DMSO | | 18.4 | 16.4 | 10.2 | DMSO |
| DMF | | 17.4 | 13.7 | 11.3 | DMF |
| NMP | | 18 | 12.3 | 7.2 | NMP |
| MeCN | | 15.3 | 18 | 6.1 | MeCN |
| THF | | 16.8 | 5.7 | 8 | THF |
| 1,4-dioxane | | 17.5 | 1.8 | 9 | 1,4-dioxane |

Based on determined HSP, solvents 1-42 (compounds 1-42) were placed in a 3D space, called Hansen space with three coordinates D, P, H (values from Table 2). This allows for the calculation of a distance between new molecules and already known compounds from the database to determine their chemically similar counterparts. Solvents that are the closest match by properties to the compounds 1-42 are likely to have similar applications and performance. However, in the case of 1-40 they could be produced sustainably from a renewable source and they probably could have a safer profile as they are derived from natural glycerol, butanetriol, acyloins (derivatives)

### Measurement of Solvatochromatic Parameters via different methods

### Kamlet-Taft parameters

Following the reference literature:
- Mortimer J Kamlet and RW Taft. "The solvatochromic comparison method. I. The. beta.-scale of solvent hydrogen-bond acceptor (HBA) basicities". In: Journal of the American chemical Society 98.2 (1976), pp. 377-383.
- Mortimer J Kamlet, Jose Luis Abboud, and RW Taft. "The solvatochromic comparison method. 6. The. pi.* scale of solvent polarities". In: Journal of the American Chemical Society 99.18 (1977), pp. 6027-6038.
- Pierre Nicolet and Christian Laurence. "Polarity and basicity of solvents. Part 1. A thermosol-vatochromic comparison method". In: Journal of the Chemical Society, Perkin Transactions 2 7 (1986), pp. 1071-1079.

Kamlet-Taft (KAT) parameters were measured by studying the wavelength of maximum absorbance (*λₘₐₓ*) of two dyes (4-nitroaniline (NA) and N,N-diethyl-4-nitroaniline (DENA).
- **Measurements of *λₘₐₓ* in conventional solvents:** The wavelength of maximum absorbance of NA and DENA in conventional solvents (n-hexane, toluene, dichloromethane, 1,2-dichloroethane, cyclohexane, DMSO and 1,4-dioxane) were measured at room temperature with a UV-VIS spectrometer. All measurements were performed in triplicates.
- **Measurements of *λₘₐₓ* in TBH, TBO, TBN, liquid state at room temperature:** Same procedure as above
- **Measurements of *λₘₐₓ* in TBO, solid state at room temperature: thermophotochromism has to be taken in to account**
For example TBO (mixture of compound 1 and 2);
The band maxima of NA and DENA at 70, 90, and 110°C. For each dye, wavelength of maximum absorbance was converted in kilokaiser and plotted against temperature in °C. Band maxima were extrapolated for a temperature of 25°C for use in subsequent calculations, in order to account for thermochromism. All measurements were performed in triplicates.

| Compound | Maximum average wavelength in NA (nm) | NA (kK) | Maximum average wavelength in DENA (nm) | DENA (kK) |
|---|---|---|---|---|
| DMSO | 389.8 | 25.654 | 412.5 | 25.586 |
| 1+2* | Extrapolation to correct for thermochromism | | | |
| 3 | 361.0 | 27.701 | 395.0 | 25.316 |
| 4 | 356.3 | 28.064 | 390.8 | 25.586 |
| 9 | 343.0 | 29.154 | 374.5 | 26.702 |

| | | | | |
|---|---|---|---|---|
| *1+2: compound 1 (76 mol%) + compound 2 (23 mol%) | | | | |

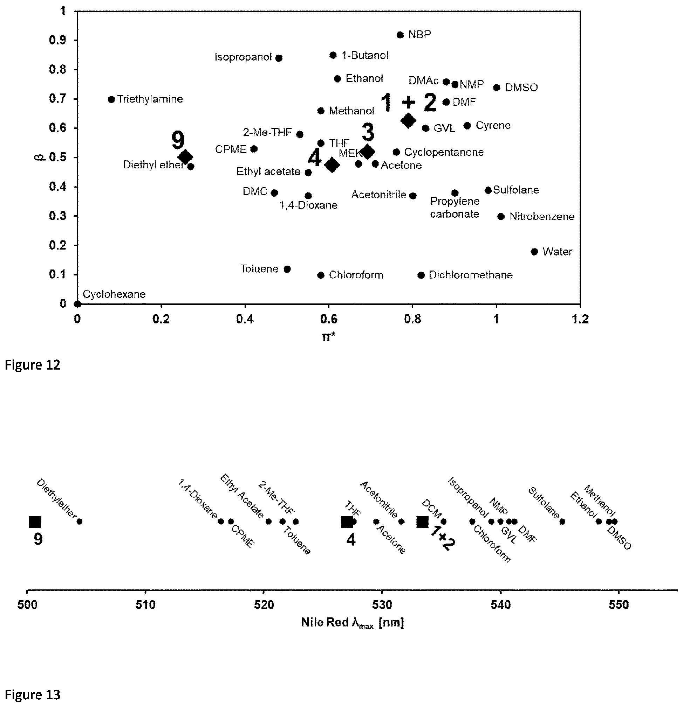

Leading to calculation of the following Kamlet taft parameters as shown in the π* vs β diagram in Figure 12 (Kamlet-Taft 2D solvent map, displaying solvent's basicity (β) and dipolarity (π*).

### Nile red

Determined following the reference literature:
- Jerry F. Deye and T. A. Berger: Anal.Chem, 1990, 62, 615-622.

***λₘₐₓ*** of Nile red, dissolved in solvents that not liquid at room temperature, e.g. TBO (mixture compound 1 and 2) was measured at 70, 90 and 110 °C. Resulting average band maxima were plotted against temperature and a linear regression was performed and used to extrapolate the band maximum at room temperature (25 °C). All measurements were performed in triplicates.

This method largely confirms the position of the numbered compounds relative to other common solvents. Figure 13 shows a representation of the Nile Red for numbered solvent compounds and other common solvents. TBO (mixture 1+2):: compound 1 (76 mol%) + compound 2 (23 mol%).

### 3. Applications

### Selected model reactions:

Investigated model reactions were all reported to show a linear solvation energy relationship (LSER) with respect to solvent KAT parameters (*vide supra,* α, β, π*). Reference works:
1. Andrew Jordan et al. "Replacement of less-preferred dipolar aprotic and ethereal solvents in synthetic organic chemistry with more sustainable alternatives". In: Chemical reviews 122.6 (2022), pp. 6749-6794.65
2. Fergal Byme et al. "2, 2, 5, 5-Tetramethyltetrahydrofuran (TMTHF): a non-polar, non-peroxide forming ether replacement for hazardous hydrocarbon solvents". In: Green chemistry 19.15 (2017), pp. 3671-3678
3. Suwiwat Sangon et al. "Direct comparison of safer or sustainable alternative dipolar aprotic solvents for use in carbon-carbon bond formation". In: Reaction Chemistry & Engineering 5.9 (2020), pp. 1798-1804

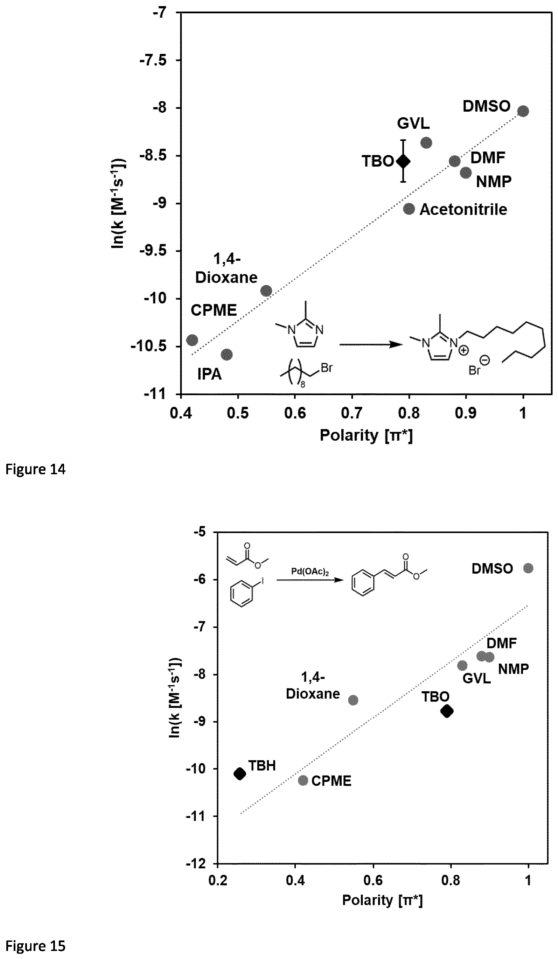

In Figures 14 to 17, the kinetics are plotted for four model reactions (Alkylation, Amidation, cross coupling, Baylis-Hillman), which support the conclusion that TBH/TBO/TBN are versatile solvents. For instance, for the case of TBO (mixture of compound 1 and 2), there is both the behavior of traditional dipolar aprotic solvents (performing well in alkylation and Baylis Hilmann coupling), while it matches more the kinetics of ethereal solvents in reactions where kinetics are favored by less polar solvents (e.g. amidation reaction), significantly surpassing the capabilities of dipolar aprotic solvents in such contexts. Furthermore, TBO benefits from the lower reactivity and an enhanced stability compared to Cyrene or GVL, making it more versatile.

### Alkylation (Menshutkin) reaction

The reaction was performed between 1,2-dimethylimidazole (0.320g, 3.23 mmol, 97% purity) and 1-bromodecane (0.65 mL, 3.08 mmol, 99% purity), in the chosen solvent (3 mL) with stirring at 70 °C. The rate of the reaction was studied in TBO (mixture of compound 1, 69 mol% and 2, 28 mol%) and eight other solvents in order to cover a range of polarities and obtain a strong correlation. The reaction was performed in 10 mL round bottom crimp cap vials.

Figure 14 (Rate constant plotted in function of relevant Kamlet-Taft parameter, for Alkylation (Menshutkin) reaction. TBO = Mixture of compound 1 (69 mol%) and compound 2 (28 mol%)) shows that TBO (mixture of compound 1 and 2) matches or outperforms the high performing dipolar aprotic reactions in the alkylation (Menshutkin) reaction.

### Cross-coupling (Heck) reaction

lodobenzene (0.69 mL, 6.00 mmol), methyl acrylate (0.54 mL, 6.00 mmol), triethylamine (0.84 mL, 6.00 mmol), and Pd(OAc)₂ (0.1 mol%) were reacted at 90 °C in 5 mL of a TBO (mixture of compound 1 and 2), a TBH (compound 9) and six other solvents to cover a range of polarities and obtain a strong correlation. The reaction was performed in 10 mL round bottom crimp cap vials.

Figure 15 (Rate constant plotted in function of relevant Kamlet-Taft parameter, for Cross-coupling (Heck) reaction. TBO = Mixture of compound 1 and compound 2) shows that TBO (mixture of compound 1 and 2) sligthly underperforms in comparison with the high performing dipolar aprotic reactions in the cross-coupling (Heck) reaction. Both TBO as well as TBH (compound 9) perform more ether-like in this model reaction.

### Amidation reaction

4-Phenylbutyric acid (0.33 g, 2 mmol) and benzylamine (0.24 mL, 2.2 mmol) were reacted at 100 °C in 4 mL of TBO (mixture of compound 1 and 2) and three other solvents to cover a range of basicities. The reaction was performed in 10 mL round bottom crimp cap vials.

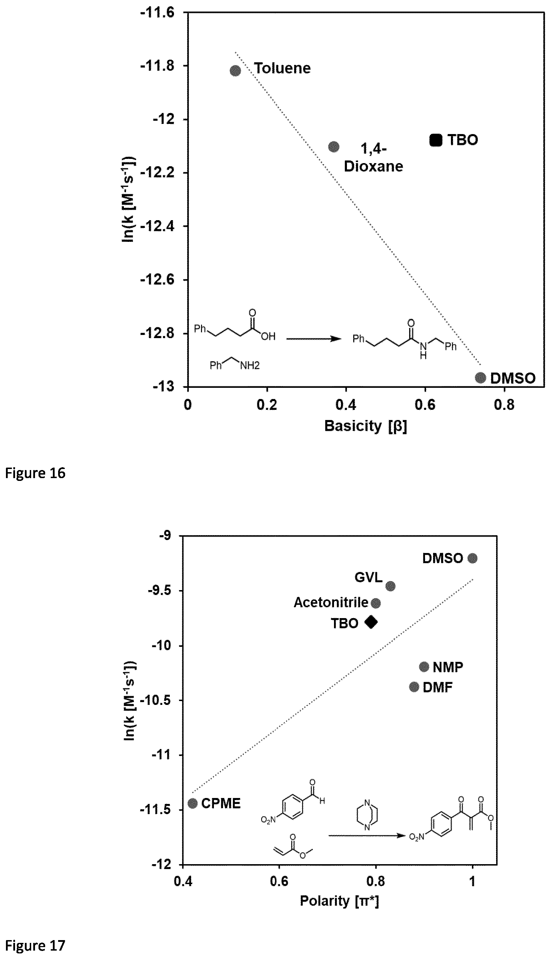

Figure 16 (Rate constant plotted in function of relevant Kamlet-Taft parameter, for Amidation reaction. TBO = Mixture of compound 1 (69 mol%) and compound 2 (28 mol%)) shows that TBO (mixture of compound 1 and 2) does not follow the LSER in the amidation reaction and merely behaves like an ether, hereby outperforming traditional dipolar solvent DMSO as this reaction typically works better for more less basic solvents.

### Baylis-Hillman reaction

4-nitrobenzaldehyde (0.30 g, 2 mmol), methyl acrylate (0.54 g, 6 mmol) and 1,4-diazabicyclo[2.2.2]octane (0.11 g, 1 mmol) were reacted at 30 °C in 2 mL of TBO (mixture of compound 1 and 2) and six other solvents in order to cover a range of polarities and obtain a strong correlation. The reaction was performed in 10 mL round bottom crimp cap vials.

Figure 17 (Rate constant plotted in function of relevant Kamlet-Taft parameter, for Baylis-Hillman reaction. TBO = Mixture of compound 1 (69 mol%) and compound 2 (28 mol%).)shows that TBO (mixture of compound 1 and 2) matches or outperforms the high performing dipolar aprotic reactions in the Bayliss Hillman reaction.

### Solid Phase Peptide synthesis (SPPS)

The increasing demand for therapeutic peptides and peptide-based drugs drives the urgent need for more sustainable synthetic methods. Solid-phase peptide synthesis (SPPS) is the method of choice to reliably synthesize peptides and small proteins. However, the current manufacturing processes compare unfavourably in terms of process mass intensity (PMI) to other modalities such as small molecules and biopharmaceuticals. The main contributor to the unfavourable PMI of SPPS is the excessive solvent usage. N,N-dimethylformamide (DMF), the most used solvent in SPPS, is classified as carcinogenic and reprotoxic. Consequently, the European Union has restricted DMF usage as of December 2023, highlighting the need for greener and safer alternatives.

### Example: Single amino acid couplings in trioxabicyclooctanes (TBO)

### General procedure for amino acid coupling:

### (Solvent = indicated below)

The amino resin (25 mg, 10 µmol, 1.0 eq.) was swelled twice with solvent (1 × 300 µL + 10µL DIPEA) for 1 min, drained, and washed with solvent (1 × 300 µL). For each coupling, a solution of Fmoc- and side-chain-protected amino acid (0.25 mL, 0.10 M in solvent, 5.0 eq.) and PyAOP (0.25 mL, 0.10 M in solvent, 5.0 eq.) was prepared. To this solution, DIPEA (17.42 µL, 0.10 mmol, 10 eq.) was added, and the solution was gently agitated at 23 °C for 1 min. The solution was then added to the resin, and the reaction was gently stirred for 20 s, then left at 23 °C for 60 min. The resin was then drained, washed with solvent (3 × 300 µL). For each deprotection step, 20% piperidine in solvent (v/v) (600 µL) was added to the resin, and the reaction was gently stirred for 20 s then left at 23 °C for 15 min. The resin was washed (1 x 300 µL solvent) and the deprotection step was repeated. The resin was then drained, then washed with solvent (3 × 300 µL) and DCM (3 × 1 mL).

Abbrevations: DIPEA: N,N-Diisopropylethylamine; PyAOP:(7-Azabenzotriazol-1-yloxy)tripyrrolidinophosphonium hexafluorophosphate); Fmoc::9-Fluorenylmethyloxycarbonyl group; DCM: Dichloro methane;

### Example 1: Single amino acid coupling & Fmoc-deprotection

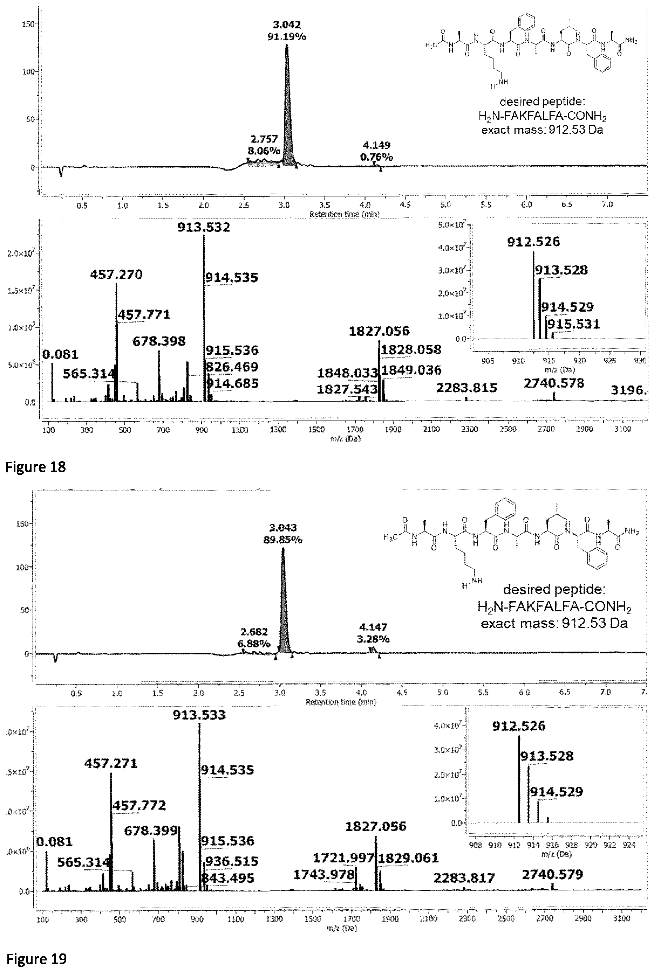

Solvent = 95:5 wt% (mixture of compound 1 + 2) : (acetonitrile)
Coupling of Phenyl alanine (F) to AKFALFA;
desired peptide: H₂N-FAKFALFA-CONH₂, exact mass: 912.53 Da:
   The above-described general procedure was followed. LC-MS results in Figure 18 (LC-MS results of single-coupling of F to AKFALFA in 95 wt% TBO 5 wt% ACN solution) show a 91.19% purity of the target peptide.

### Example 2: Single amino acid coupling & Fmoc-deprotection

Solvent = Compound 3
Coupling of Phenyl alanine (F) to AKFALFA;
desired peptide: H₂N-FAKFALFA-CONH₂, exact mass: 912.53 Da:
   The above-described general procedure was followed. LC-MS results in Fig. 19 (LC-MS results of single-coupling of F to AKFALFA in compound 3) show a 89.95% purity of the target peptide.

### Example 3: DMF-free synthesis of 5-mer peptide

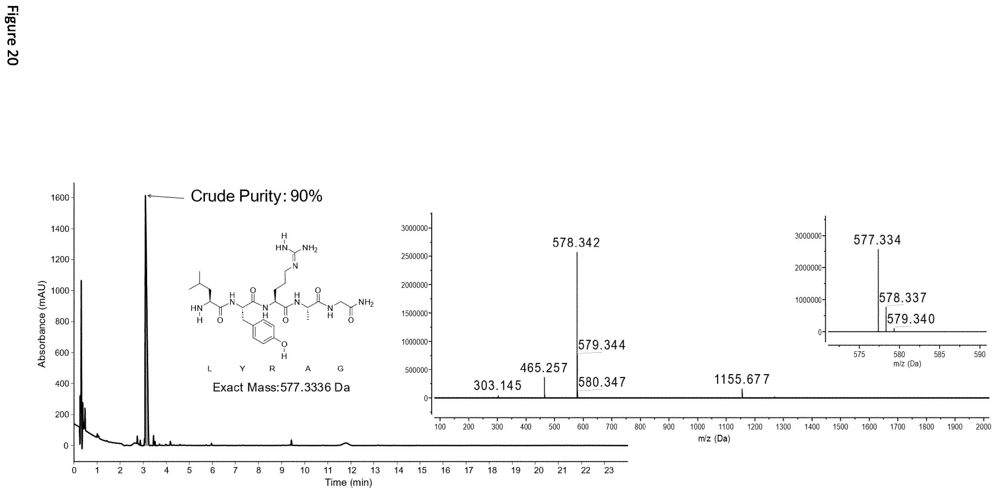

Solvent = 95:5 wt% (mixture of compound 1 + 2) : (acetonitrile)
A 5-mer peptide was then synthesized, largely following the above-described protocol for coupling of all amino acids.
Desired peptide: LYRAG, exact mass: 577.3336 Da
UPLC + MS results in Figure 20 (UPLC + MS results for LYRAG synthesis in 95 wt% TBO 5 wt% ACN solution ) show a crude purity for LYRAG of 90%.

### Example 4: Comparison of JR-10 peptide compared to DMF

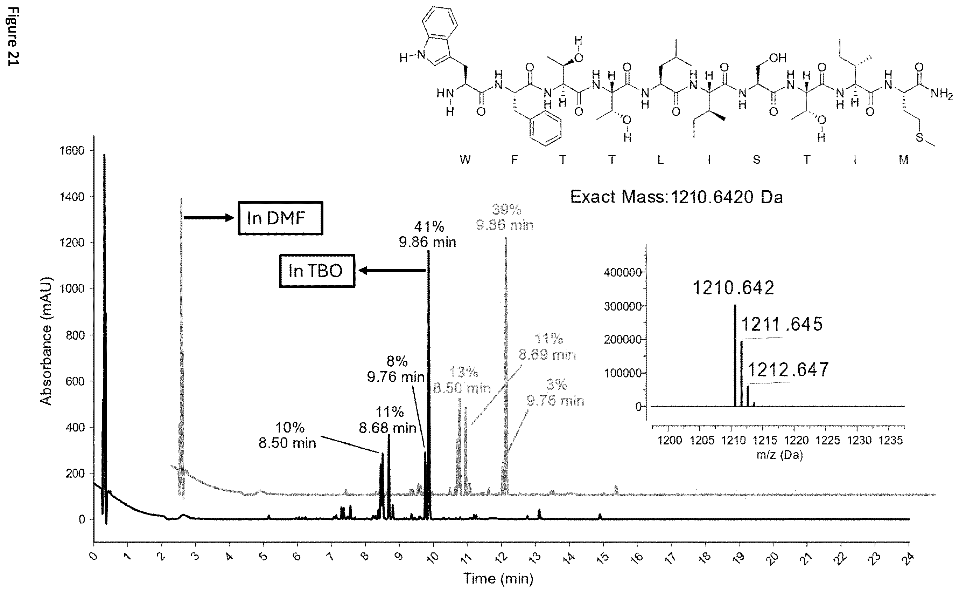

Solvent = 95:5 wt% (mixture of compound 1 + 2) : (acetonitrile)
JR-10, a challenging to synthesize peptide (well-known aggregating sequence) was synthesized both in solvent, as well as in traditional DMF as a comparison, largely following the above-described protocol for coupling of all amino acids.
Desired peptide: JR-10, exact mass: 1210.6420 Da
UPLC + MS results in Figure 21 (UPLC + MS results for JR-10 synthesis in 95 wt% TBO 5 wt% ACN solution in comparison to in DMF) show a crude purity of 41 % (solvent) compared to 39% (DMF)

### Rendering Solid TBHs / TBO / TBNs liquid at room temperature

Some of the presented solvents are solids at room temperature. Other solvents such as Ethylene carbonate (m.p. 35 °C) and sulfolane (m.p. 27.5°C) also show this key characteristic. For example, TBO (mixture of compound 1 + 2 (m.p. 54 °C) is a gel/solid at room temperature. This can have advantages (e.g. for transport). However, there are several methods to render solids into liquids at room temperatures for solvent use, conveniently. For instance, TBO (mixture of compound 1 + 2) turns liquid at room temperature with following methods:

### Preparing deep eutectic solvents

Deep eutectic solvents (DESs) are eutectic mixtures composed of a hydrogen bond donor and a hydrogen bond acceptor with a significantly lower melting point compared to those of the individual components. The mechanism behind this phenomenon involves the formation of strong hydrogen bonds between the components, which leads to a decrease in the melting point of the mixture. TBH, TBO and TBN contain 3 oxygen atoms in their structure, and are therefore, in general, strong hydrogen bond donor acceptors. Therefore, as shown in the following table, hydrogen bond donors may form eutectics with (mixture of) compound 1 + 2

**(Table 3)**

| **Hydrogen bond donor** | **M.p. (°C)** | **M.p. (°C) of the 1:1 Molar Mixture with TBO** |
|---|---|---|
| **HBD** | **of the Individual compound** | **compound 1+2 (75 mol% 25 mol% ratio))** |
| Phenol | 40 | Liquid at -18 °C |
| Resorcin | 110 | Liquid at 4 °C, solid at -18 °C |
| Catechol | 105 | Liquid at 4 °C, solid at -18 °C |
| Propyl Guaiacol | 16 | Liquid at -18 °C |

### Preparing TBH/TBO/TBN Blends

Solvent blending is a common practice used to obtain a new solvent system with attractive properties that differ from those of the individual solvents. This method has recently been used to lower the viscosity of Cyrene by blending it with 2-MeTHF, GVL, DMF, and acetonitrile in 50-90 wt%. 50 wt% blend of Cygnet. (solid at room temperature) and Cyrene was successfully used as a solvent system for the preparation of polymeric membranes.

It was found that adding, e.g., 5 wt% of common other solvents can render **TBO (mixture of compound 1 + 2)** liquids at room temperature, and demonstrate interesting solvatochromatic parameters:

**Table with Hanssen parameters, and the absorption band maxima for the two dyes (NA, Nitroaniline) and (DENA, dinitroanline), allowing for calculation of the Polarity π* and basicity β (Kamlet Taft parameters)**

| **Solvent blend (wt%)** | **HSP** | | | **Kamlet Taft** | | | **NA** | **DENA** |
|---|---|---|---|---|---|---|---|---|
| | **δD** | **δP** | **δH** | **α** | **β** | **π*** | **λₘₐₓ (nm)** | |
| X = TBO* 95: ACN 5 | 17.4 | 9.2 | 7.2 | n/m | 0.48 | 0.83 | 366.0 | 402.0 |
| Y = TBO* 95: 1,3-dioxolane 5 | 17.6 | 8.4 | 7.4 | 0 | 0.51 | 0.79 | 365.0 | 400.0 |
| Z = TBO* 95 : 2-MeTHF 5 | 17.9 | 8.4 | 7.6 | 0 | 0.55 | 0.77 | 365.3 | 399.0 |

| | | | | | | | | |
|---|---|---|---|---|---|---|---|---|
| *TBO = mixture of compound 1 (69 mol%) and compound2 (28 mol%) | | | | | | | | |

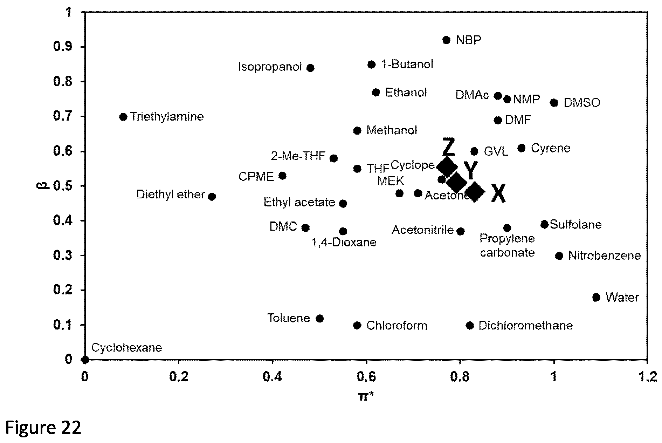

Figure 22 shows the Kamlet-Taft 2D solvent map, displaying solvent's basicity (β) and dipolarity (π*) for solvent blends X, Y, Z.

### Polymer dissolution

### Solution polymerization reactions in TBH/TBO/TBN

Industrially, numerous synthetic methods and processing techniques for polymers involve the use of organic solvents. The main function herein is to solubilize reagents such as monomers and polymers, maintain a homogeneous mixture and influence rate constants. Also generally, a solvent that can solubilize the polymer is necessary in order to achieve high molecular-weight polymers.

For example, Atom Transfer Radical Polymerzation (ATRP) are commonly performed in polar aprotic solvents such as DMF, DMSO, THF, NMP, DMAc. Given the non-benign character of these solvents, recently there has been a growing interest in non-toxic bioderived alternatives that possess similar properties reflected by the Hanssen solubility parameters, such as 2-MeTHF and Cyrene.

Another example, biocatalysis (Lipases) for polyester (Polycondensation) synthesis. The traditional solvent choice for polycondensation reactions is environmentally hazardous diphenyl ether (DPE). In general, ether solvents have proven to be effective for these reactions due to their non-reactivity towards towards lipases, and their medium polarity, especially favourable. Unfortunately, many traditional ether solvents are toxic.

While the proposed solvents all have their own issues (high boiling point, viscous, reacivity), their hanssen solubility parameters and kamlet taft parameters are similar to many of the covered TBO/TBH/TBN compounds which supports the conclusion that the compounds used in the context of the invention can also be covered for polymer synthesis methods.

| **Solvent structures** | **HSP** | | | **Kamlet Taft** | | |
|---|---|---|---|---|---|---|
| | **δD** | **δP** | **δH** | **α** | **β** | **π*** |
| 2-MeTHF | 16.9 | 5 | 4.3 | 0 | 0.58 | 0.53 |
| 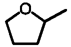 | | | | | | |
| Cyrene | 18.8 | 10.6 | 6.9 | 0 | 0.61 | 0.93 |
| 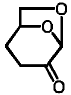 | | | | | | |
| DPX | 16.4 | 6.1 | 4.5 | 0 | 0.63 | 0.68 |
| 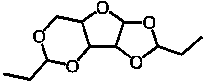 | | | | | | |
| 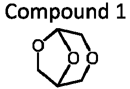 | 17.9 | 8.6 | 7.8 | 0 | 0.63 | 0.79 |

Sources for values of table above:
- Green Chem., 2012,14, 1245-1259
- Chem. Commun., 2014,50, 9650-9652
- Chemsuschem volume 18, issue 6, e202401877, 2025

### Polymer processing (e.g. solution spinning)

For solution spinning for fiber formation, the polymer is first dissolved in a solvent, forming a spinning solution (sometimes called a "dope"). The spinning solution then undergoes dry, wet, dry-jet wet, gel, or electrospinning techniques. Polymeric materials do not dissolve instantaneously, and the dissolution is controlled by either disentanglement of the polymeric chains or by the diffusion of the chains through a boundary layer adjacent to the polymer-solvent interface.

Polylactic acid (PLA) for example, is soluble in dioxane, acetonitrile, chloroform, methylene chloride, 1,1,2-trichloroethane and dichloro acetic acid, while it is only partially soluble in ethyl benzene, toluene, acetone and tetrahydrofuran, only when heated to boiling temperature. PLA is insoluble in water, alcohols and linear hydrocarbons.

Poly(methyl methacrylate) (PMMA) is generally soluble in polar organic solvents such as ethyl acetate, cyclohexanone, chloroform, tetrahydrofuran, trichloroethylene, N,N-dimethylformamide.

The advantage here is that TBH, TBO and TBN could replace the solvents as mentioned above, often halogenated, toxic and/or restricted.

### Example:

As a proof of concept, a solvent blend consisting of 95 wt% of TBO (compound 1 (77 mol%) + 2 (22 mol%)) and 5 wt% acetonitrile (referred to as "Solvent blend") was used to dissolve two traditional polymers.

### Polylactic acid (PLA) procedure:

PLA granules (125.6 mg, Natureworks 4032D, Mw = 207'000)) were put in a vial together Solvent blend (1 mL) and put in an ultrasonic bath for 30 minutes at 60°C, after which the solution had turned into a fully transparent liquid, which stayed stable after cooling down to room temperature.

### Poly(methylmethacrylate) (PMMA) procedure:

PMMA powder (250.0 mg , Mw = 15'000) was put in a vial together with Solvent blend (1 mL) and put in an ultrasonic bath for 5 minutes at 60°C, after which the cloudy solution had turned into a fully transparent liquid, which stayed stable after cooling down to room temperature.

## Claims

1. Use of one or more types of a bridged, bicyclic compound selected from a trioxabicycloheptane, a trioxabicyclooctane, a trioxabicyclononane, and from alkylated and/or alkoxylated derivatives of any of these compounds, as solvent(s).

2. The use in accordance with claim 1, wherein the one or more types of the bridged, bicyclic compound is/are selected from 2,5,7-trioxabicyclo[2.2.1]heptane, 3,6,8-trioxabicyclo[3.2.1]octane, 2,5,7-trioxabicyclo[2.2.2]octane, 3,7,9-trioxabicyclo[4.2.1]nonane, 3,8,9-trioxabicyclo[4.2.1]nonane, 3,7,9-trioxabicyclo[3.3.1]nonane, and from alkylated and/or alkoxylated derivatives of any of these compounds.

3. The use in accordance with claim 1 or 2, wherein the alkylated and/or alkoxylated derivatives are derivatives carrying one or more substituents independently selected from methyl, ethyl, methoxy and ethoxy attached to one or more carbon ring members of the bridged, bicyclic ring system of the trioxabicycloheptane, the trioxabicyclooctane or the trioxabicyclononane.

4. The use in accordance with any one of claims 1 to 3 wherein the one or more types of the bridged, bicyclic compound is/are used as solvent(s) comprised by a solvent formulation, and
wherein the solvent formulation comprises one or more types of the bridged, bicyclic compound mixed with at least one further solvent which is not a bridged, bicyclic compound selected from a trioxabicycloheptane, a trioxabicyclooctane, a trioxabicyclononane, and from alkylated and/or alkoxylated derivatives of any of these compounds, or
wherein the solvent formulation comprises a eutectic mixture of the one or more types of the bridged, bicyclic compound and at least one hydrogen bond donor compound.

5. The use in accordance with claim 4, wherein the solvent formulation comprises the one or more types of the bridged, bicyclic compound at total concentration of 20 wt% or more, based on the total weight of the solvent formulation.

6. The use in accordance with any one of claims 1 to 5, wherein the one or more types of the bridged, bicyclic compound is/are used as solvent(s) for a chemical reaction, as process solvent(s), as solvent(s) in polymer production and/or processing, as extraction solvent(s), as solvent(s) for purifying a substance, as solvent(s) for cleaning, as solvent(s) for the formulation and/or application of an active agent, or as solvent(s) for the storage of a substance.

7. The use in accordance with claim 6, wherein the chemical reaction is selected from a cross-coupling reaction, an alkylation reaction, a hydrogenation reaction, an enzymatic reaction, an amidation reaction and an esterification reaction.

8. The use in accordance with any one of claims 1 to 6, wherein the one or more types of the bridged, bicyclic compound is/are used as solvent(s) in peptide synthesis.

9. The use in accordance with any one of claims 1 to 6, wherein the one or more types of the bridged, bicyclic compound is/are used as solvent(s) in solution spinning.

10. A method for the preparation of a solution or dispersion, comprising a step of dissolving or dispersing a substance in a single solvent or in a solvent formulation, wherein the single solvent is a bridged, bicyclic compound selected from a trioxabicycloheptane, a trioxabicyclooctane, a trioxabicyclononane, and from alkylated and/or alkoxylated derivatives of any of these compounds and wherein the solvent formulation comprises at least one type of a bridged, bicyclic compound selected from a trioxabicycloheptane, a trioxabicyclooctane, a trioxabicyclononane, and from alkylated and/or alkoxylated derivatives of any of these compounds as solvent(s).

11. The method in accordance with claim 10, wherein the preparation of the solution or dispersion forms part of a process for carrying out a chemical reaction, a process for the production of a polymer, a process for the preparation of a polymer coating, a polymer film or a polymer membrane, a solution spinning process, a peptide synthesis, or a process for the formulation of an active agent.

12. A solution or dispersion, wherein a substance is dissolved or dispersed in a single solvent or in a solvent formulation, wherein the single solvent is a bridged, bicyclic compound selected from a trioxabicycloheptane, a trioxabicyclooctane, a trioxabicyclononane, and from alkylated and/or alkoxylated derivatives of any of these compounds and wherein the solvent formulation comprises at least one type of a bridged, bicyclic compound selected from a trioxabicycloheptane, a trioxabicyclooctane, a trioxabicyclononane, and from alkylated and/or alkoxylated derivatives of any of these compounds as solvent(s).

13. The solution or dispersion in accordance with claim 12, wherein the dissolved or dispersed substance comprises at least one of the following (i) to (iv):
(i) at least one type of polymer, such as a polyacrylate, a polymethacrylate (such as poly(methyl methacrylate)), a polyether, a polyurea, a polyacetal, a polyolefin, a polysulfone, a polyamide, a polyimide, a polyester (such as lactic acid or glycolic acid), polystyrene, polyacrylonitrile, polyvinylchloride, polyvinylpyrrolidone, polyvinylacetate, a polycarbonate, a polyurethane, an epoxy resin, a cellulose derivative, or a copolymer combining appropriate units of two or more of these exemplary polymers;
(ii) at least one type of a monomer suitable for the formation of a polymer or copolymer as referred to in (i);
(iii) at least one type of organic chemical reactant, preferably an amino acid or a derivative thereof carrying one or more protecting groups, e.g. a protecting group for an amino group;
iv) at least one type of an active agent, preferably an agrochemical active agent.

14. A solvent formulation comprising one or more types of a bridged, bicyclic compound selected from a trioxabicycloheptane, a trioxabicyclooctane, a trioxabicyclononane, and from alkylated and/or alkoxylated derivatives of any of these compounds, at a total concentration of 20 wt% or more, based on the total weight of the solvent formulation, which solvent formulation is either
(i) a solvent mixture wherein the one or more types of a bridged, bicyclic compound is/are mixed with at least one further solvent which is not a bridged, bicyclic compound selected from a trioxabicycloheptane, a trioxabicyclooctane, a trioxabicyclononane, and from alkylated and/or alkoxylated derivatives of any of these compounds, or
(ii) a eutectic mixture wherein the one or more types of a bridged, bicyclic compound are mixed with at least one hydrogen bond donor compound.

15. A method for the preparation of a trioxabicyclooctane or of an alkylated derivative thereof, said method comprising
a) a step wherein a compound of formula (S1) 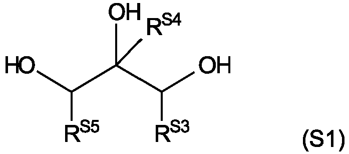 wherein R^{S3}, R^{S4} and R^{S5} are independently hydrogen or methyl,
is reacted with a compound of formula (S2A) or a compound of formula (S2B) 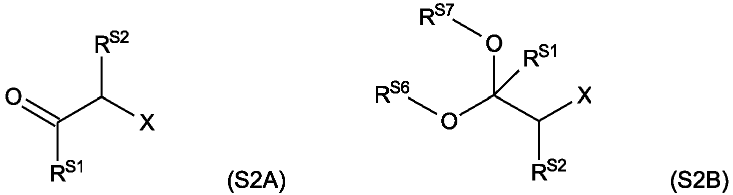 wherein
X is Cl or Br,
R^{S1} and R^{S2} are independently hydrogen or methyl,
R^{S6} and R^{S7} are C1-C3 alkyl;
to obtain a mixture of isomer compounds (S3T), (S3C), (S4T) and (S4C), wherein X, R^{S1}, R^{S2}, R^{S3}, R^{S4} and R^{S5} are defined as above, 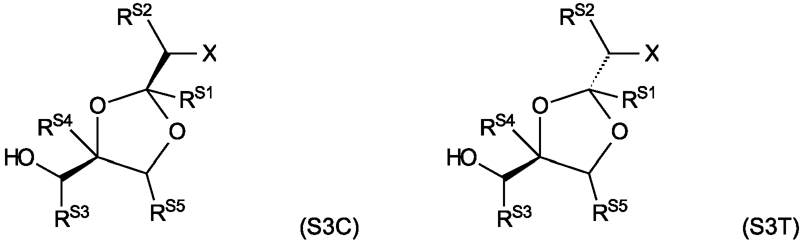 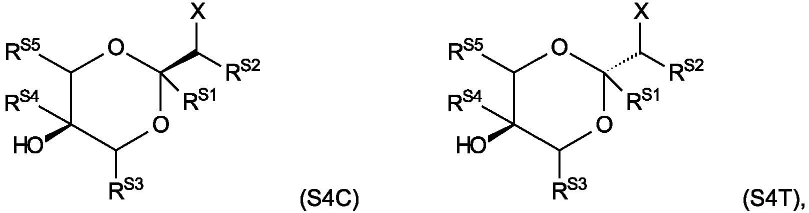 and
b) a step wherein the compounds of formula (S3C) and (S4C) are subjected to intramolecular ether formation in the presence of NaOH or KOH in an amount of 1 to 2.70 molar equivalents, relative to the total amount of compounds of formula (S3C) and (S4C), in water,
to obtain the compounds of formula (S5) and (S6), wherein R^{S1}, R^{S2}, R^{S3}, R^{S4} and R^{S5} are as defined above:
